(19) Europäisches Patentamt
European Patent Office
Office européen des brevets

(11) **EP 4 624 457 A1**

(12) **EUROPEAN PATENT APPLICATION**

(43) Date of publication:
**01.10.2025 Bulletin 2025/40**

(21) Application number: **24305488.9**

(22) Date of filing: **29.03.2024**

(51) International Patent Classification (IPC):
***C07D 221/20*** *(2006.01)* ***A61P 13/12*** *(2006.01)*
***A61P 25/00*** *(2006.01)* ***A61P 35/00*** *(2006.01)*
***A61K 31/435*** *(2006.01)*

(52) Cooperative Patent Classification (CPC):
**A61P 25/00; A61P 13/12; A61P 35/00; C07D 221/20**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Centre National de la Recherche Scientifique 75016 Paris (FR)**
- **Commissariat à l'Energie Atomique et aux Energies Alternatives 75015 Paris (FR)**
- **INSTITUT FRANCAIS DE RECHERCHE POUR L'EXPLOITATION DE LA MER - IFREMER 29280 Plouzané (FR)**
- **INSTITUT PASTEUR 75724 Paris Cedex 15 (FR)**

(72) Inventors:
- **ARAOZ, Romulo 91190 Gif-sur-Yvette (FR)**
- **DELEPIERRE, Muriel 92120 Montrouge (FR)**
- **BIGNON, Jérôme 91190 Gif-sur-Yvette (FR)**
- **HESS, Philipp 44240 La Chapelle-sur-Erdre (FR)**
- **SÉCHET, Véronique 44311 Nantes (FR)**

(74) Representative: **Novagraaf Technologies**
**Bâtiment O2**
**2, rue Sarah Bernhardt**
**CS90017**
**92665 Asnières-sur-Seine Cedex (FR)**

(54) **INGRILIMINE AND DERIVATIVES, INVOLVEMENT IN NEURODEGENERATIVE DISEASES**

(57) Compound of formula (I) or of its pharmaceutically acceptable form selected among salt, prodrug, crystal form, stereoisomer, tautomer, hydrate and solvate thereof:

EP 4 624 457 A1

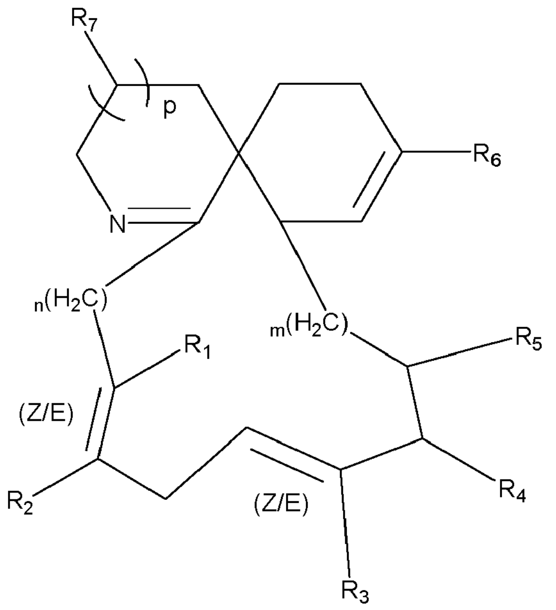

(I)

- wherein the groups $R_1$, $R_2$ and $R_3$ represent independently a hydrogen or an aliphatic chain being at least selected among one of the following: linear, branched, cyclic, substituted and unsubstituted chain;
- wherein the groups $R_4$ and $R_5$ represent independently a substituent comprising at least one of the atom or function selectionned from -F; -Cl; -Br; -I; $-NO_2$; -CN; $-CF_3$; -OH and -SH;
- wherein $R_6$ represents a substituent comprising at least one of the atom or function selected from -F; -Cl; -Br; -I; $-NO_2$; -CN; $-CF_3$; -OH; -SH; thioether; amine; carbonyl; an aliphatic chain selected among a $C_1$-$C_6$ alkyl, preferably an alkene group; -C(O)-OH; and an ester;
- wherein the one, or two groups, $R_7$ represent independently a hydrogen or a methyl;
- wherein n and m are integers, independently selected among 1 to 3; and
- wherein p is an integer selected among 1 and 2.

## Description

## FIELD OF THE INVENTION

The present invention relates to novel spiroimines developed for their use as a drug, and more particularly, but not exclusively, to treat the diseases involving nicotinic acetylcholine receptors in animals or in human being, in particular the α7 nicotinic acetylcholine receptors.

In what follows, the numbers between brackets (**[ ]**) refer to the list of references provided at the end of the description.

## BACKGROUND OF THE INVENTION

The emerging cyclic imines toxins (CiTXs) of global distribution across coastal environments that are produced by dinoflagellates move fast up the human food chain through shellfish. The CiTX family includes 44 compounds: 7 gymnodimines (A-E, 12-methylgymnodimine and 16-desmethyl gymnodimine D), 16 spirolides (A-D, G-I, 13-desmethyl spirolide-C, 27-Hydroxy-13-desmethyl spirolide-C, 13,19-didesmethyl spirolide-C, 20-hydroxy-13,19-didesmethyl spirolide- C, 27-hydroxy-13,19-spirolide-C, 27-Oxo-13,19-didesmethyl spirolide-C, 13-desmethyl spirolide-D, 20-hydroxy-13,19-didesmethyl spirolide-D and 20-methyl spirolide-G), 8 pinnatoxins (A-H), 3 pteriatoxins (A-C), 6 prorocentrolides (A-B, 4- hydroxyprorocentrolide, 9,51-dihydro prorocentrolide, 30-sulfate prorocentrolide and 14-O-acetyl-4-hydroxyprorocentrolide), 1 spiroprorocentrimine, 2 *portimines* (A and B), and 1 *kabirimine,* (Aráoz et al. (2020) Cyclic imine toxins survey in coastal european shellfish samples: Bioaccumulation and mode of action of 28-O-palmitoyl ester of pinnatoxin-G. First report of portimine-A bioaccumulation. Harmful Algae, 98 **[1]**). The spiroimine moiety is the major feature characterizing cyclic imine toxins. The exception to this rule is prorocentrolides in which a hexahydroisoquinoline group replaces the spiroimine motif, (Torigoe et al. (1988) Prorocentrolide, a toxic nitrogenous macrocycle from a marine dinoflagellate, Prorocentrum-lima. Journal of the American Chemical Society, 110:7876-7877 **[2]**). Portimines display a 5-membered cyclic imine ring; *gymnodimines*, prorocentrolides, spiroprorocentrimines and *kabirimine* present a 6-membered imine ring, while spirolides, pinnatoxins and pteriatoxins display a 7-membered imine ring. Cyclic imine toxins are fast acting neurotoxins that, when administered intraperitoneally or by gavage at lethal doses, kill mice by respiratory arrest within minutes following blockade of neuromuscular nicotinic acetylcholine receptors (nAChR). The mode of action of several cyclic imines on nAChRs has been characterized, namely: *gymnodimine*-A, 13- desmethyl spirolide- C, 13,19-didesmethyl spirolide-C, 20-methyl spirolide-G, pinnatoxin-A, pinnatoxin-G, prorocentrolide-A and *portimine,* (Lamoise et al. (2017) Physico-chemical and functional characterization of portimine purified from Vulcanodinium rugosum strain IFR-VRU-01. In "Marine and Fresh-Water Harmful Algae". Proceedings of the 17th International Conference on Harmful Algae (Proença, L.A.O. and Hallegraeff, G. eds) pp. 126-129 ISSHA and IOC/UNESCO, Florianapolis **[3];** Molgó et al. (2017) Cyclic imine toxins from dinoflagellates: a growing family of potent antagonists of the nicotinic acetylcholine receptors. Journal of Neurochemistry, 142:41-51 **[4]**). They are potent antagonists of muscular and neuronal nicotinic acetylcholine receptors with pinnatoxin-A displaying picomolar affinities towards the α7 nicotinic acetylcholine receptor subtype (Aráoz et al. (2011) Total synthesis of pinnatoxins A and G and revision of the mode of action of pinnatoxin A. Journal of the American Chemical Society 133(27):10499-10511 **[5]**). In contrast, prorocentrolide and *portimine* show micromolar affinities towards the α7 nicotinic acetylcholine receptor subtype (Lamoise et al. (2017) [3], Stivala et al. (2015) Synthesis and biology of cyclic imine toxins, an emerging class of potent, globally distributed marine toxins. Natural Product Reports, 32(3):411-435 **[6]**) (Table1).

**Table 1.** Antagonistic activity of CiTX (Two-Electrodes Voltage Clamp electrophysiology).

| CiTX | nAChR subtype (nM; 95% CI) | | |
|---|---|---|---|
| | α12β1γδ (Torpedo) | α7 (human) | α4β2 (human) |
| *Gymnodimine*-A | 2.8 (1.9-4.1) | n.d. | 0.9 (0.6-1.2) |
| 13-desmethyl spirolide-C | 0.51 (0.4-0.6) | 0.18 (0.16-0.21) | 3.9 (2.9-5.1) |
| 13,19-didesmethyl spirolide-C | 0.20 (0.16-0.26) | 0.25 (0.24-0.27) | 6.26 (4.7-8.3) |
| 20-methyl spirolide-G | 0.36 (0.29-0.45) | 0.48 (0.15-1.4) | 2.1 (1.4-3.1) |
| Pinnatoxine-A | 5.53 (4.5-6.8) | 0.107 (0.086-0.132) | 30.4 (19.4-47.5) |
| Pinnatoxine-G | 3.82 (2.99-4.88) | 5.06 (3.84-6.67) | 4.90 (3.97-6.06) |
| Palmitate PnTX-G | 0.97 (0.75-2.13) | n.d. | n.d. |
| Prorocentrolide | 185.7 (165.07-209.05) | 1660 (1641-1680) | n.d. |
| *Portimine*-A | 11920 (6968-20380) | 21160 (12520-35770) | n.d. |

(continued)

| CiTX | nAChR subtype (nM; 95% CI) | | |
|---|---|---|---|
| | α12β1γδ (Torpedo) | α7 (human) | α4β2 (human) |
| *Ingrilimine* | n.d. | 2.29 (1.02-5.14) | n.d. |

Nicotinic acetylcholine receptors are allosteric, ligand-gated cation-selective pentameric transmembrane proteins belonging to the Cysteine Loop Ligand-gated Ion Channel Superfamily. The binding of acetylcholine triggers the opening of the receptor's channel for the passage of $Na^+$ and $K^+$ ions (Albuquerque et al. (2009) Mammalian nicotinic acetylcholine receptors: From structure to function. Physiological Reviews, 89(1):73-120 **[7],** Taly et al. (2009) Nicotinic receptors: allosteric transitions and therapeutic targets in the nervous system. Nature reviews Drug discovery, 8(9):733-750 **[8]).** Nicotinic acetylcholine receptors display a high structural and functional diversity. Muscular nicotinic acetylcholine receptors mediate fast neurotransmission at the neuro-muscular junction ensuring respiration and muscle contraction essential for physical mobility and daily life (Mukund et al. (2020) Skeletal muscle: A review of molecular structure and function, in health and disease. Wiley Interdisciplinary Reviews-Systems Biology and Medicine, 12(1) **[9]**). At the central Nervous System, neuronal nicotinic acetylcholine receptors modulate the release of neurotransmitters participating in fundamental aspects of synaptic plasticity involved in attention, learning, memory and development. Neuronal α4β2 and α7 nicotinic acetylcholine receptor subtypes are major pharmacological targets for a series of neurodegenerative diseases including Alzheimer disease, Parkinson disease, schizophrenia, epilepsy, autism and addiction (Bertrand et al. (2015) Therapeutic potential of alpha7 nicotinic acetylcholine receptors. Pharmacological reviews, 67(4):1025-1073 **[10],** Dani and Bertrand (2007) Nicotinic acetylcholine receptors and nicotinic cholinergic mechanisms of the central nervous system. In: Annual Review of Pharmacology and Toxicology, 47: 699-729 **[11]**). Thus, the cholinergic hypothesis behind Alzheimer disease has provided over the years the theoretical support for the development of novel therapeutics (Bartus (2000) On neurodegenerative diseases, models, and treatment strategies: Lessons learned and lessons forgotten a generation following the cholinergic hypothesis. Experimental Neurology, 163(2):495-529 **[12]**). Of the four FDA approved front-line drugs for Alzheimer disease treatment in 2017, donepezil and rivastigmine inhibit acetylcholinesterase, galantamine modulates allosterically α7 nicotinic receptors and inhibits acetylcholinesterase, and memantine inhibits NMDA receptors and blocks α7 nAChR. A fifth available medication combines rivastigmine and memantine. Although none of these drugs alters the course of the disease, treated Alzheimer disease patients performed better on memory and thinking tests delaying and/ or slowing the worsening of cognitive impairment of treated patients (McDade and Bateman (2017) Stop Alzheimer's before it starts. Nature, 547(7662):153-155 **[13]**). In addition, non-neuronal nicotinic acetylcholine receptors are ubiquitously expressed in the human body from epithelial cells, to primary immune organs such as the bone marrow, thymus and macrophages performing a host of diverse functions in almost all non-neuronal mammalian cells (Wessler and Kirkpatrick (2008) Acetylcholine beyond neurons: the non-neuronal cholinergic system in humans. British Journal of Pharmacology, 154(8):1558-1571 **[14]**). On the one hand, it was shown that the α7 nicotinic acetylcholine is responsible for the proliferative, pro-angiogenic and pro-metastatic effects of nicotine in lung cancer (Wang and Hu (2018) 7 nicotinic acetylcholine receptors in lung cancer. Oncology Letters, 16(2):1375-1382 **[15].** Currently, antagonists of α7 nicotinic acetylcholine receptors antagonists are considered to be potentially useful anticancer drugs for therapeutic purposes. On the other hand, mitochondrial α7 nicotinic acetylcholine receptors may regulate early events of mitochondria-driven apoptosis (Resende and Adhikari (2009) Cholinergic receptor pathways involved in apoptosis, cell proliferation and neuronal differentiation. Cell Communication and Signaling 2009, 7:20 **[16]**). The interest of working with cyclic imine toxins and neurodegenerative diseases has been reported (Alonso et al. (2013) Benefit of 13-desmethyl spirolide C treatment in triple transgenic mouse model of Alzheimer Disease: Beta-Amyloid and neuronal markers improvement. Current Alzheimer Research, 10(3):279-289 **[17],** Alonso et al. (2011) The cholinergic antagonist gymnodimine improves a beta and tau neuropathology in an in-vitro model of Alzheimer Disease. Cellular Physiology and Biochemistry, 27(6):783-794 **[18]**). Gymnodimine-A and 13-desmethyl spirolide-C ameliorated glutamate induced neurotoxicity decreasing β-amyloid plaques deposition, thus improving Alzheimer disease neuronal biomarkers and increasing acetylcholine levels in *in-vitro* and *in-vivo* Alzheimer disease-animal models, see patent application: Botana et al. (2011) Use of gymnodimine, analogues and derivatives for the treatment and/or prevention of neurodegenerative diseases associated with Tau and B-amyloid. WO2011039394 **[19],** and, Botana et al. (2012) Use of a spirolide, analogues and derivatives for treating and/or preventing pathological conditions linked to the tau and beta-amyloid proteins. US20120258956A1 **[20]**). Further, 20-methyl spirolide-G showed neuroprotective effects against the 1-methyl-4-phenylpyridinium ion that induces the degeneration of human neuronal stem cells *in-vitro,* increasing the synthesis of acetylcholine and of α7 nAChR (Boente-Juncal et al. (2018) In vitro effects of chronic spirolide treatment on human neuronal stem cell differentiation and cholinergic system development. ACS Chemical Neuroscience, 9(6):1441-1452 **[21]**).

*Portimine,* described in the patent application WO2014189393 (Hampton et al. (2014) Bioactive compounds.

WO2014189393 **[22]**), is a spiro-linked five-membered cyclic imine possessing strong cytotoxic and apoptotic properties. *Portimine,* however, is not very active on α7 nicotinic acetylcholine receptors. Hence, there is still strong need to develop novel molecules, with robust cytotoxic and apoptotic properties, and with high affinity toward α7 nicotinic acetylcholine receptors.

## BRIEF DESCRIPTION OF THE DRAWINGS

**Figure 1** represents the chemical structure of *ingrilimine* according to compound (IIIa) formula.

**Figure 2** represents the cytotoxic activity of *ingrilimine.* A2780 ovarian and HT29 colorectal carcinoma cells were grown in RPMI 1640 supplemented with 10% fetal calf serum (FCS) and 1% glutamine. U87-MG glioblastoma cells were grown in Dulbecco minimal essential medium containing 4.5 g/L glucose, 10% FCS and 1% glutamine at 37 °C in a humidified atmosphere containing 5% $CO_2$. For $IC_{50}$ determination, the cells were seeded in 96-well plates ($3 \times 10^3$ cells/well) containing 90 μL of growth medium. After 24 h of culture, the cells were treated with *ingrilimine* at 10 different final concentrations. Cell viability was determined by luminescence after 72 h incubation.

(a) Effect of *ingrilimine* on A2780 ovarian carcinoma cells. (b) Effect of *ingrilimine* on U87-MG glioblastoma cells. (c) Effect of *ingrilimine* on U87-MG glioblastoma cells

Figure 3a-3f represent the mitochondrial dysfunction provoked by *ingrilimine.* HCT116 cells were treated with *ingrilimine* in the range of 10-500 nm for 24h and 48h. Sector Q normal cells that accumulated the JC-1 dye (fluorescence emission 590 nm). Sector R shows the apoptotic cells that accumulated JC-1 in which the fluorescence emission shifted from 590 nm to 530 nm due to mitochondrial membrane depolarization. (a) Non-treated cells incubated with the carrier (DMSO) for 24 h. (b) HCT116 cells incubated with 10 nN *ingrilimine* for 24 h. (c) HCT116 cells incubated with 250 nM ingrilimine for 24 h. (d) Non-treated cells incubated with the carrier (DMSO) for 48 h. (e) HCT116 cells incubated with 10 nN *ingrilimine* for 48 h. (f) HCT116 cells incubated with 250 nM ingrilimine for 48. Changes in fluorescence emission were recorded by flow cytometry using the JC-1 mithocondrial membrane potential-dependent fluorescent dye. NT = non-treated cells; IngX = *ingrilimine.*

Figure 4a-4f represents phosphatidylserine exposure provoked by ingrilimine. A2780 cancer cells were incubated with *ingrilimine* in the range of 10-500 nM before staining with FITC-Annexin V and propidium iodide. Quadrants D1 and D2 with dead or necrotic cells, quadrant D3 with normal cells and quadrant D4 with apoptotic cells following phosphatidylserine exposure. (a) Non-treated A2780 cells incubated with the carrier (DMSO) for 24h. (b) A2780 cells incubated with 10 nM *ingrilimine* for 24 h. (c) A2780 cells incubated with 250 nM *ingrilimine* for 24 h. (d) Non-treated A2780 cells incubated with the carrier (DMSO) for 48 h. (e) A2780 cells incubated with 10 nM *ingrilimine* for 48 h. (f) A2780 cells incubated with 250 nM *ingrilimine* for 48. Phosphatidylcholine exposure in A2780 cell was quantified by flow cytometry using FITC-Annexin V. Dead or necrotic cells were detected using the vital dye propridium iodide. NT = non-treated cells; IngX = *ingrilimine.*

**Figure 5** represents the antagonistic activity of *ingrilimine* (named "IngX" in the figure) and *portimine* (named "Port A" in the figure) against nicotinic acetylcholine receptors recorded by two-electrodes voltage clamp electrophysiology on *Xenopus laevis* oocytes. Clamped *Xenopus laevis* oocytes having incorporated the muscle $(\alpha1)_2\beta\gamma\delta$ nAChR subtype from *Torpedo marmorata* (filled squares), or expressing the neuronal α3β2 nAChR subtype from rat (filled triangles), or expressing the human neuronal α7 nAChR subtype (filled circles) were exposed to *portimine* (100 nM - 1 mM) for 45 seconds. *Portimine* inhibited the acetylcholine-evoked current in the micromolar range against the three nAChRs subtypes tested (TABLE 1). The $IC_{50}$ of *portimine* against the α7 nAChR subtype was 21160 nM. On the contrary, the antagonistic activity of *ingrilimine* against the human α7 nAChR subtype (filled diamonds) was very strong ($IC_{50}$ = 2.3 nM). *Ingrilimine* was ~10 000-times more potent than *portimine* against the human α7 nAChR subtype (Table 1).

**Figure 6** represents the dual activity of *ingrilimine* on the human α7 nAChR subtype. A *Xenopus laevis* oocyte expressing the human α7 nAChR subtype was exposed to 100 μM acetylcholine triggering the immediate opening of the receptor's channel (black bold line) followed by the very-fast closure of the channel (microseconds) that became insensitive to acetylcholine during the rest of the 5 seconds exposure (desensitization). When a *Xenopus laevis* oocyte expressing the human α7 nAChR subtype was exposed first to 10 nM *ingrilimine* for 45 seconds and then to a mixture (Mix) containing 10 nM *ingrilimine* and 100 μM acetylcholine, the receptor's channel was closed denoting a strong antagonistic activity of the tested toxin against the α7 nAChR subtype (dotted line). Further, when a *Xenopus laevis* oocyte expressing the human α7 nAChR subtype was exposed to 10 μM *ingrilimine,* the receptor's channel immediately opened for a very-short time denoting that ingilimine behaves as an agonist of the human α7 nAChR subtype in the micromolar range (black line). Notice that upon incubation of the oocyte to 10 μM *ingrilimine,* the receptor's channel did not open when the oocyte was exposed to a mixture containing 10 μM *ingrilimine* and 100 μM acetylcholine. *Ingrilimine* displayed a dual activity against the human α7 nAChR subtype: at nanomolar concentrations, it behaves as an antagonist, while at micromolar concentrations, it behaves as an agonist of α7 nAChR subtype.

**Figure 7** represents the dual activity of *ingrilimine* (IngX) on the human α7 nAChR subtype. At nanomolar concentrations, *ingrilimine* behaves as an antagonist of the human α7 nAChR subtype with an $IC_{50}$ of 2.3 nM (black line) ($IC_{50}$ defined as the half-maximal inhibitory concentration needed for closing the receptor's channel). At micromolar concentrations, *ingrilimine* behaves as an agonist of the human α7 nAChR subtype (dotted line). The $EC_{50}$ (the concentration of agonist that provokes the opening of the receptor's channel halfway between the baseline (Bottom) and maximum response (Top)) of *ingrilimine* upon α7 nAChR could not be obtained because of the higher amount of toxin needed for this purpose).

**DEFINITIONS**

The term "pharmaceutically acceptable form", as used herein, denotes any pharmaceutically acceptable salt, hydrate, ester or salt of such hydrate or ester, of a compound, or any other adduct or derivative which, upon administration to a patient, can provide (directly or indirectly) a compound as otherwise described herein, or a metabolite or residue thereof. Pharmaceutically acceptable forms thus include among other prodrugs. "Hydrates" are compounds formed by the union of water and another substance, usually resulting in a neutral body, such as certain crystallized salts.

A "prodrug" is a derivative of a compound, usually with significantly reduced pharmacological activity, which contains an additional moiety that is susceptible to removal *in-vivo* yielding the parent molecule as the pharmacologically active species. An example of a prodrug is an ester which is cleaved *in-vivo* to yield a compound of interest. Prodrugs of a variety of compounds, and materials and methods for derivatizing the parent compounds to create the pro-drugs, are known and may be adapted to the present invention.

The term "stable", as used herein, preferably refers to compounds which possess stability sufficient to allow manufacture and which maintain the integrity of the compound for a sufficient period to be detected and preferably for a sufficient period to be useful for the purposes detailed herein. The term "stability" can refer to the ability to resist degradation, to persist in a given environment, and/or to maintain a particular structure.

The term "aliphatic", as used herein, includes both saturated and unsaturated, straight chain (*i.e.*, unbranched) or branched aliphatic hydrocarbons, which are optionally substituted with one or more functional groups. As will be appreciated by one of ordinary skill in the art, "aliphatic" is intended herein to include, but is not limited to, alkyl, alkenyl, alkynyl moieties. Thus, as used herein, the term "alkyl" includes straight and branched alkyl groups. An analogous convention applies to other generic terms such as "alkenyl", "alkynyl" and the like. Furthermore, as used herein, the terms "alkyl", "alkenyl", "alkynyl" and the like encompass both substituted and unsubstituted groups.

The term "alkyl" includes also "cycloalkyl". The term "cycloalkyl", as used herein, refers for example to cyclopropyl, cyclobutyl, cyclopentyl, cyclohexyl, cycloheptyl and the like, which, as in the case of aliphatic, heteroaliphatic or heterocyclic moieties, may optionally be substituted. An analogous convention applies to other generic terms such as "cycloalkenyl", "cycloalkynyl" and the like.

The term "$C_1$-$C_6$ alkyl", as used herein, refers to a substituted or unsubstituted, linear, branched (and/or eventually totally or partially cyclic), saturated monovalent radical consisting solely of carbon and hydrogen atoms, having from one to six carbon atoms, and having a free valence "-" at one end of the radical.

Illustrative aliphatic groups for straight and branched alkyl groups include, but are not limited to, for example, methyl, ethyl, n-propyl, isopropyl, allyl, n-butyl, sec-butyl, isobutyl, tert-butyl, n-pentyl, sec-pentyl, isopentyl, tert-pentyl, n-hexyl, sec-hexyl, moieties and the like, which again, may bear one or more substituents. Alkenyl groups include, but are not limited to, for example, ethenyl, propenyl, butenyl, 1-methyl-2-buten-l-yl, and the like. Representative alkynyl groups include, but are not limited to, ethynyl, 2-propynyl (propargyl), 1-propynyl and the like.

The term "diene", as used herein, refers to compounds according to the IUPAC definition, meaning compounds that contain two fixed double bonds (usually assumed to be between carbon atoms). Dienes in which the two double-bond units are linked by one single bond are termed conjugated, e.g. $CH_2$=CH-CH=$CH_2$ buta-1,3-diene. Dienes in which the double bonds are adjacent are called cumulative, e.g. $CH_3$-CH=C=$CH_2$ buta-1,2-diene. Those in which one or more of the unsaturated carbon atoms is replaced by a heteroatom may be called "heterodienes". The term "diene group", as used herein, refers to a diene molecule as a monovalent radical having a free valence "-" at one end.

The term "alicyclic", as used herein, refers to compounds which combine the properties of aliphatic and cyclic compounds and include but are not limited to cyclic, or polycyclic aliphatic hydrocarbons and bridged cycloalkyl compounds, which are optionally substituted with one or more functional groups. As will be appreciated by one of ordinary skill in the art, "alicyclic" is intended herein to include, but is not limited to, cycloalkyl, cycloalkenyl, and cycloalkynyl moieties, which are optionally substituted with one or more functional groups. Illustrative alicyclic groups thus include, but are not limited to, for example, cyclopropyl, -$CH_2$-cyclopropyl, cyclobutyl, -$CH_2$-cyclobutyl, cyclopentyl, -$CH_2$-cyclopentyl-n, cyclohexyl, -$CH_2$-cyclohexyl, cyclohexenylethyl, cyclohexanylethyl, norborbyl moieties and the like, which again, may bear one or more substituents.

The term "heteroaliphatic" refers to aliphatic moieties in which one or more carbon atoms in the main chain have been substituted with a heteroatom. Thus, a heteroaliphatic group refers to an aliphatic chain which contains one or more

oxygen, sulfur, nitrogen, phosphorus or silicon atoms, *i.e.,* in place of carbon atoms.

The term "heteroalicyclic", "heterocycloalkyl" or "heterocyclic", refers to compounds that combine the properties of heteroaliphatic and cyclic compounds and include but are not limited to saturated and unsaturated mono- or polycyclic heterocycles such as morpholino, pyrrolidinyl, furanyl, thiofuranyl, pyrrolyl etc., which are optionally substituted with one or more functional groups, as defined herein. In certain embodiments, the term "heterocyclic" refers to a non-aromatic 5-, 6- or 7- membered ring or a polycyclic group, including, but not limited to, a bi- or tri-cyclic group comprising fused six-membered rings having between one and three heteroatoms independently selected from oxygen, sulfur and nitrogen. Wherein (i) each 5-membered ring has 0 to 2 double bonds and each 6-membered ring has 0 to 2 double bonds, (ii) the nitrogen and sulfur heteroatoms may optionally be oxidized, (iii) the nitrogen heteroatom may optionally be quaternized, and (iv) any of the above heterocyclic rings may be fused to an aryl or heteroaryl ring. Representative heterocycles include, but are not limited to, pyrrolidinyl, pyrazolinyl, pyrazolidiny1, imidazolinyl, imidazolidinyl, piperidinyl, piperazinyl, oxazolidiny1, isoxazolidinyl, morpholinyl, thiazolidinyl, isothiazolidinyl, and tetrahydrofuryl.

In general, the term "aromatic moiety", as used herein, refers to stable substituted or unsubstituted unsaturated mono- or polycyclic hydrocarbon moieties having preferably 3-14 carbon atoms, comprising at least one ring satisfying the Huckel rule for aromaticity. Examples of aromatic moieties include, but are not limited to, phenyl, indanyl, indenyl, naphthyl, phenanthryl and anthracyl.

In general, the term "heteroaromatic moiety", as used herein, refers to stable substituted or unsubstituted unsaturated mono-heterocyclic or polyheterocyclic moieties having preferably 3-14 carbon atoms, comprising at least one ring satisfying the Huckel rule for aromaticity. Examples of heteroaromatic moieties include, but are not limited to, pyridyl, quinolinyl, dihydroquinolinyl, isoquinolinyl, quinazolinyl, dihydroquinazolyl, and tetrahydroquinazolyl.

The terms "alkoxy" (or "alkyloxy"), and "thioalkyl" refers to an alkyl group, as previously defined, attached to the parent molecular moiety through an oxygen atom ("alkoxy") or through a sulfur atom ("thioalkyl"). Examples of alkoxy groups include, but are not limited to, methoxy, ethoxy, propoxy, isopropoxy, n-butoxy, tert-butoxy, neopentoxy and n-hexoxy. Examples of thioalkyl groups include, but are not limited to, methylthio, ethylthio, propylthio, isopropylthio, n-butylthio, and the like.

The terms "hydroxyl" as used herein refers to a group having the structure -OH, and the term "hydroxyl protecting group", as used herein, unless otherwise indicated, refers to a substituent that is commonly employed to block or protect a hydroxyl on the compound thereby protecting its functionality while allowing for the reaction of other functional groups on the compound. Non-exclusive examples of a hydroxyl protecting group include: acyl groups (e.g., formyl, acetyl, chloroacetyl, trichloroacetyl, O-nitrophenylacetyl, O-nitrophenoxyacetyl, trifluoroacetyl, acetoacetyl, 4-chlorobutyryl, isobutyryl,O-nitrocinnamoyl, picolinoyl, acylisothiocyanate, aminocaproyl, benzoyl, and the like), acyloxy groups (e.g., 1-tert-butyloxycarbonyl (Boc), methoxycarbonyl, 9-fluorenyl-methoxycarbonyl, 2,2,2-trifluoroethoxycarbonyl, 2-trimethylsilylethxoycarbonyl, vinyloxycarbonyl, allyloxycarbonyl, 1,1-dimethyl-propynyloxycarbonyl, benzyloxycarbonyl, p-nitrobenzyloxycarbony, 2,4-dichlorobenzyloxycarbonyl, and the like), diphenylmethane, silyl ether (e.g., tert-butyldimethylsilyl ether, abbreviated by "TBS") and benzylcarbamates. A "hydroxyl group deprotection" as used herein refers to the chemical reaction involving a deprotecting agent to remove the substituent that had been precedently used to protect the hydroxyl.

The terms "halo" and "halogen" as used herein refer to an atom selected from fluorine, chlorine, bromine and iodine.

The term "halogenated" denotes a moiety having one, two, or three halogen atoms attached thereto.

The term "haloalkyl" denotes an alkyl group, as defined above, having one, two, or three halogen atoms attached thereto and is exemplified by such groups as chloromethyl, bromoethyl, trifluoromethyl, and the like.

The term "ester", as used herein, does not substantially differ from the common meaning of this term in the art, and refers to a moiety of structure -C(O)-ORx, wherein Rx is preferably a substituted or unsubstituted aliphatic, alicyclic, heteroaliphatic, heteroalicyclic, aryl or heteroaryl moiety.

The term "lactone", as used herein, does not substantially differ from the common meaning of this term in the art, and refers to a structure according to the definition of IUPAC: a cyclic ester of hydroxy carboxylic acids, containing a 1-oxacycloalkan-2-one structure, or analogues having unsaturation or heteroatoms replacing one or more carbon atoms of the ring. The term "lactone group" as used herein refers to a lactone molecule as a monovalent radical having a free valence "-" at one end.

The term "carbonyl", as used herein, does not substantially differ from the common meaning of this term in the art, and refers to a moiety of structure -C(O)-.

The term "imino", as used herein, does not substantially differ from the common meaning of this term in the art, and refers to a moiety of structure $-C(=NR_X)R_Y$, wherein $R_X$ is hydrogen or an optionally substituted aliphatic, alicyclic, heteroaliphatic, heteroalicyclic, aryl or heteroaryl moiety; and $R_Y$ is an optionally substituted aliphatic, alicyclic, heteroaliphatic, heteroalicyclic, aryl or heteroaryl moiety.

"Therapeutically effective amount" refers to an amount of formulation, composition, or reagent in a pharmaceutically acceptable carrier or a physiologically acceptable salt of an active compound that is of sufficient quantity to

ameliorate the undesirable state of the patient, animal, material, or object so treated. "Ameliorate" refers to a lessening of the detrimental effect of the disease state or disorder, in the receiver of the treatment.

"Pharmaceutical agent or drug" refers to a chemical compound or composition capable of inducing a desired therapeutic or prophylactic effect when properly administered to a subject.

"Pharmaceutical excipient" refers to any chemical compound in a pharmaceutical composition other than the active pharmaceutical ingredient.

"Pharmaceutical carrier" refers to any chemical compound capable of interacting with, and enhancing the properties, of active pharmaceutical ingredients.

An "agonist" is a molecule that interacts with and activates a receptor.

An "antagonist" is a molecule that interacts with a receptor and blocks or diminishes the physiological effect of the natural ligand

A "E configuration" is a disposition of substituents from each part of an atomic bound, typically as in an alkene configuration, two groups of higher priority are on opposite sides of the double bond according to Cahn-Ingold-Prelog priority rules

BRIEF DESCRIPTION OF THE INVENTION

The present invention relates to a compound having the following formula I :

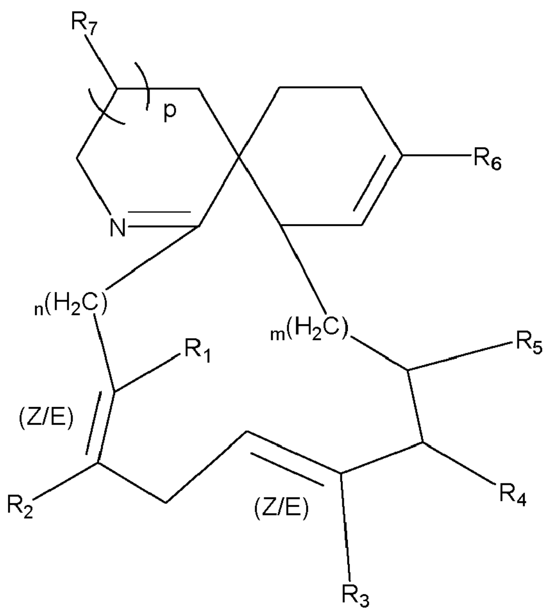

(I)

- wherein the groups $R_1$, $R_2$ and $R_3$ represent independently a hydrogen or an aliphatic chain being at least selected among one of the following: linear, branched, cyclic, substituted and unsubstituted chain;

- wherein the groups $R_4$ and $R_5$ represent independently a substituent comprising at least one of the atom or function selectionned from -F; -Cl; -Br; -I; $-NO_2$; -CN; $-CF_3$; -OH and -SH;

- wherein $R_6$ represents a substituent comprising at least one of the atom or function selected from -F; -Cl; -Br; -I; $-NO_2$; -CN; $-CF_3$; -OH; -SH; thioether; amine; carbonyl; an aliphatic chain selected among a $C_1$-$C_6$ alkyl, preferably an alkene group; -C(O)-OH; and an ester;

- wherein the one, or two groups, $R_7$ represent independently a hydrogen or a methyl;

- wherein n and m are integers, independently selected among 1 to 3; and

- wherein p is an integer selected among 1 and 2.

The compound according to the invention exclude the following molecules : The CiTX family includes 44 compounds: 7 gymnodimines (A-E, 12-methylgymnodimine and 16-desmethyl gymnodimine D), 16 spirolides (A-D, G-I,

13-desmethyl spirolide-C, 27-Hydroxy-13-desmethyl spirolide-C, 13,19-didesmethyl spirolide-C, 20-hydroxy-13,19-didesmethyl spirolide- C, 27-hydroxy-13,19-spirolide-C, 27-Oxo-13,19-didesmethyl spirolide-C, 13-desmethyl spirolide-D, 20-hydroxy-13,19- didesmethyl spirolide-D and 20-methyl spirolide-G), 8 pinnatoxins (A-H), 3 pteriatoxins (A-C), 6 prorocentrolides (A-B, 4-hydroxyprorocentrolide, 9,51-dihydro prorocentrolide, 30-sulfate prorocentrolide and 14-O-acetyl-4-hydroxyprorocentrolide), 1 spiroprorocentrimine, 2 *portimines* (A and B), and 1 *kabirimine,* (Aráoz et al. (2020)

The present invention relates in addition to a pharmaceutically acceptable form of compound (I), preferably selected among a salt, prodrug, crystal form, stereoisomer, tautomer, hydrate and solvate thereof. The compound according to the following list: *portimines, gymnodimines, pinnatoxins, spirolides, pteriatoxines, prorocentrolides, spiroprorocentimines,* and *kabirimine* are unambiguously and strictly excluded from the compounds of formula (I).

Preferably, the aliphatic chain of the groups $R_1$, $R_2$ and $R_3$ is selected among a $C_1$-$C_{12}$ alkyl, and preferably a $C_1$-$C_6$ alkyl such as a methyl, an ethyl or a propyl, and preferably a methyl.

Preferably, the groups $R_4$ and $R_5$ represent independently a -OH.

Preferably, the compound according to formula (I) is selected among at least one of the following compounds (Ia) to (Ij):

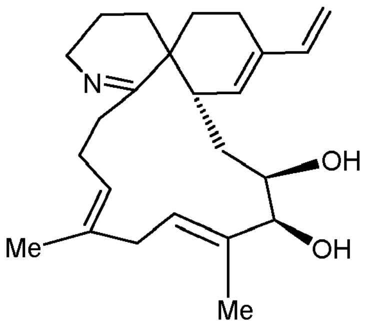

(Ia)

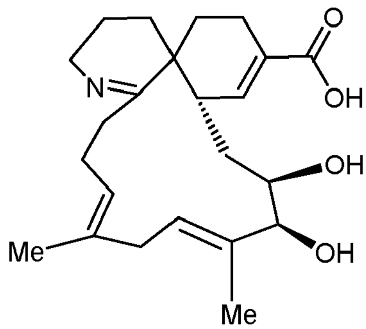

(Ib)

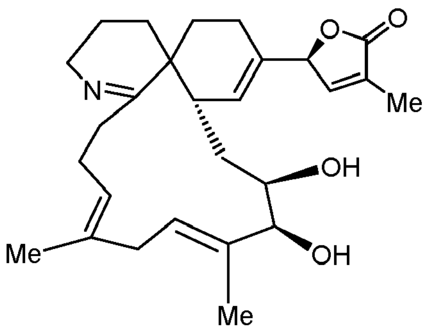

(Ic)

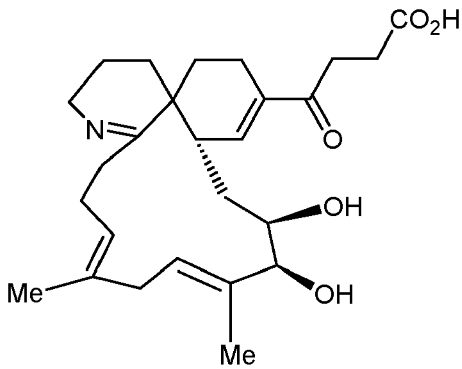

(Id)

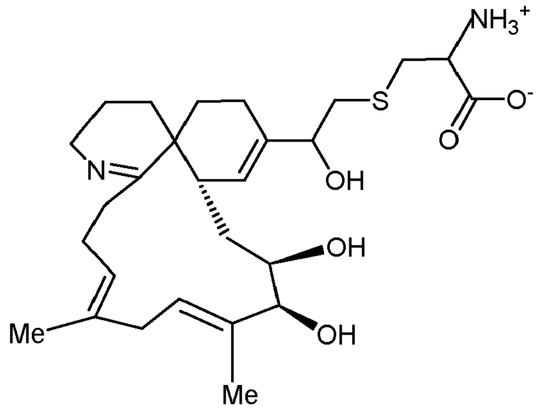

(Ie)

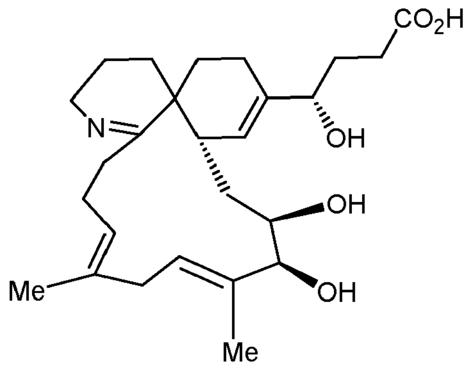

(If)

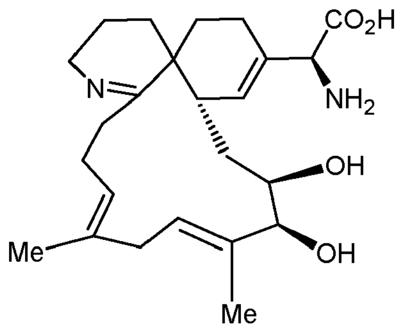

(Ig)

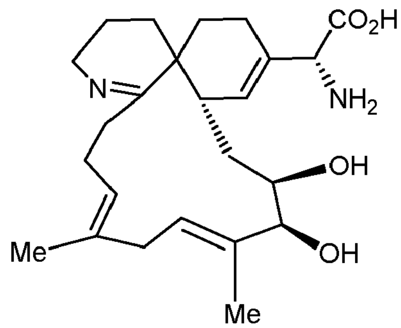

(Ih)

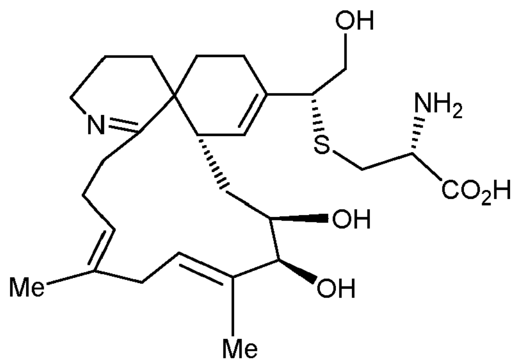

(Ii)

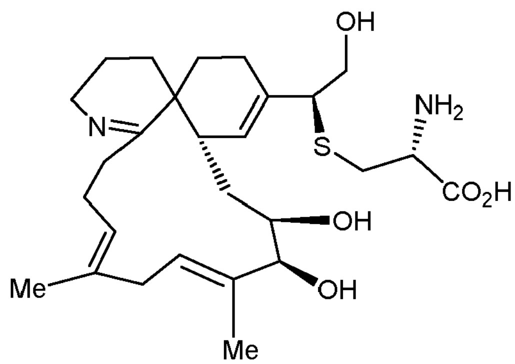

(Ij)

Preferably, the compound according to the present invention is of formula (II):

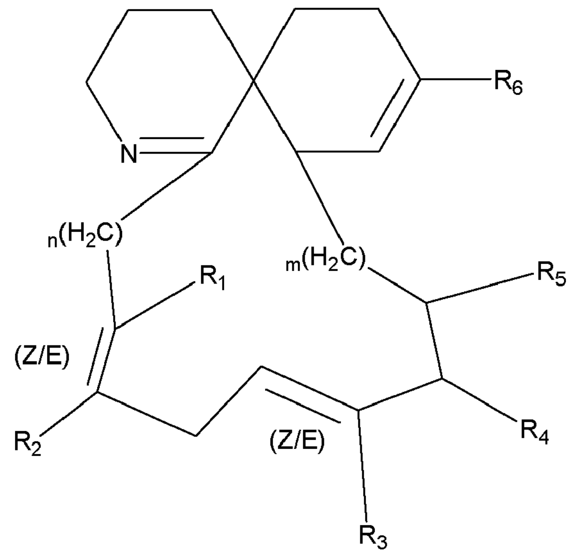

(II)

and wherein the groups $R_1$, $R_2$, $R_3$, $R_4$, $R_5$ and $R_6$; and m, n and p are as defined precedently in the context of the present invention, $R_1$ is preferably a hydrogen and $R_2$ and $R_3$ are independently preferably a $C_1$-$C_6$ alkyl, preferably a methyl. Preferably, the groups $R_4$ and $R_5$ represent independently a -OH.

Preferably, the compound according to the present invention is an *ingrilimine* having the following formula (III) :

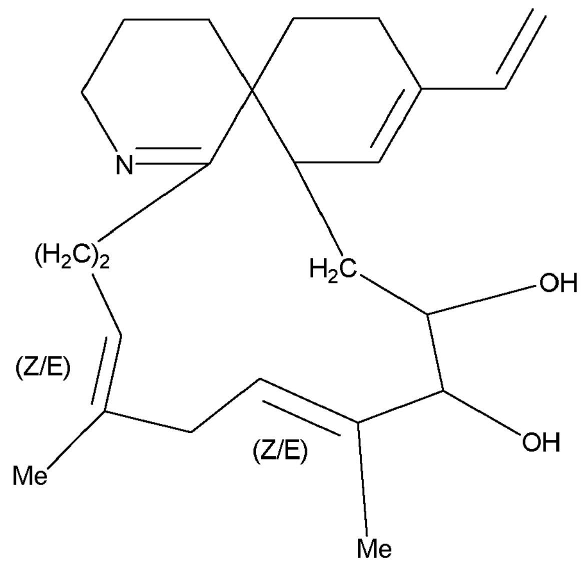

(III)

Preferably, the compound according to the present invention is selected among at least one of the followings compounds (IIIa) and (IIIb) or a mixture:

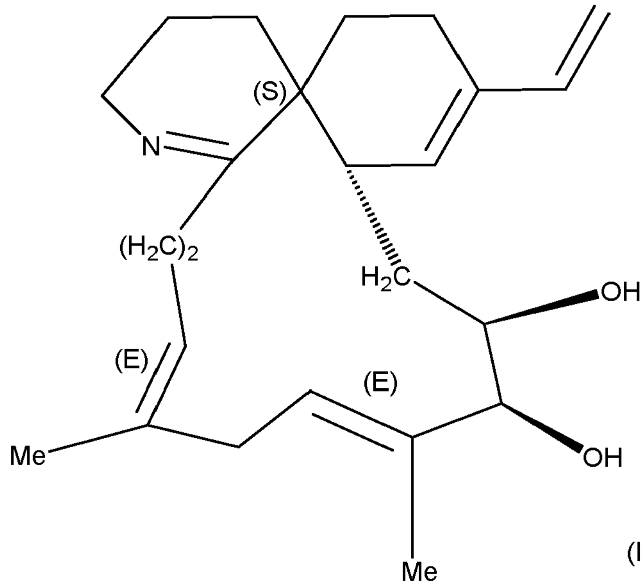

(IIIa)

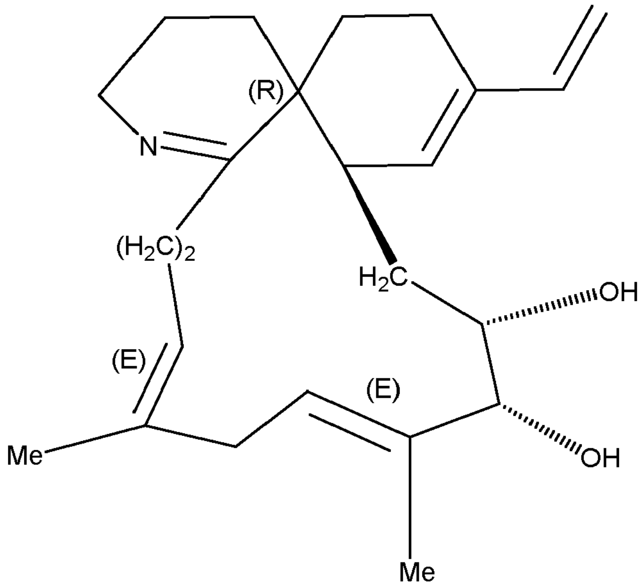

(IIIb)

Preferably, the compound according to the present invention is the compound (IIIa).

Preferably, in the precedently presented compounds among (I), (II) and/or (III), the double bond bearing the $R_1$ group, preferably a hydrogen, and the $R_2$ group, preferably a methyl, is in the E configuration; and the double bond bearing the $R_3$ group, preferably a methyl, is in the E configuration.

The present invention relates also to any compound as precendently presented in the context of the invention, and selected among at least one of the compounds (I), (II), (III), (IIIa) and (IIIb), for use as a medicament.

Preferably, said compounds (I), (II), (III), (IIIa) and/or (IIIb) is for use in the treatment of Chronic Kidney Disease (CDK).

Preferably, said compounds (I), (II), (III), (IIIa) and/or (IIIb) is for use in the treatment of at least one of the neurodegenerative diseases selected among Alzheimer disease, Parkinson disease, Schizoprenia, Epiliepsy, Autism and Addiction, preferably Autism.

Preferably, said compounds (I), (II), (III), (IIIa) and/or (IIIb) is for use in the treatment of a cancer, preferably lung cancer.

Preferably, said compounds (I), (II), (III), (IIIa) and/or (IIIb) is for the use selected among one of the followings: as an $\alpha_7$ nicotinic acetylcholine receptor antagonist and as an $\alpha_7$ nicotinic acetylcholine receptor agonist. Advantageously, said $\alpha_7$ nicotinic acetylcholine receptor is a human $\alpha_7$ nicotinic acetylcholine receptor.

The present invention relates also to a pharmaceutical composition comprising the compound as precendently presented in the context of the invention, said compound being preferably dissolved in a nanomolar concentration, and

more preferably said compound is dissolved in a concentration from 1 to 10 nanomole/L in respect of the total volume of said pharmaceutical composition.

Alternaltely, the present invention relates also to a pharmaceutical composition comprising the compound as precendently presented in the context of the invention, said compound being preferably dissolved in a micromolar concentration, and more preferably said compound is dissolved in a concentration from 1 to 10 micromole/L in respect of the total volume of said pharmaceutical composition.

The pharmaceutical composition according to the present invention, comprising a pharmaceutically acceptable carrier, preferably used as an excipient.

Besides, the present invention relates to a therapeutic method involving any of the compounds or composition precedently presented within the scope of the present invention or a pharmaceutical acceptable form thereof, preferably for use in the treatment of a subject suffering of any affection as precedently presented within the context of the present invention.

The present invention relates preferably to a method of treating any diseases as precedently reported in the scope of the present invention, comprising once a day the administration of a dose of the compound or the pharmaceutical composition according to the invention.

Preferably the pharmaceutical composition according to the invention is administrated by one of the following routes of administration: oral; sublingal; intravenous; intramuscular; subcutaneous and/or intrathecal.

Besides, the present invention relates to the use of any compound precedently presented within the scope of the present invention or a pharmaceutical acceptable form thereof for the manufacture of a medicament, preferably for the treatment of a subject suffering of any affection as precedently presented within the context of the present invention. Said medicament is preferably for use in the treatment of an animal or a human, preferably a human.

The present invention relates also to a non-therapeutic use of compound or composition as precedently described in the context of the invention, or a pharmaceutical acceptable form thereof, in a non-human mammal, preferably the mouse, in a healthy subject not suffering from any syndrome or condition.

## DETAILED DESCRIPTION OF CERTAIN EXEMPLARY EMBODIMENTS OF THE INVENTION

### Part I. Purification and chemical study of the molecules

### Material and methods:

The ethanolic extract of *Vulcanodinium rugosum* IFR-VRU-01 from which, *ingrilimine* and portimine were purified, was kindly provided by P. HESS (Ifremer, Nantes). All physical data for *ingrilimine* and *portimine,* including $^{1}H$ and $^{13}C$ NMR spectroscopy, high-resolution mass spectrometry (HRMS), HPLC, and infrared absorption spectroscopy (IFR) showed both compounds to be of high purity. HRMS analysis was performed using a QExactive Focus Orbitrap LC-MS/MS system (Thermo Scientific). NMR experiments were recorded on either an 800 MHz Avance NEO (Bruker Biospin, Billerica, MA, USA) spectrometer with an 18.1 tesla magnetic field or a 600 MHz Avance III HD spectrometer with a 14.1 tesla magnetic field for proton frequency. Both NMR units were equipped with a cryogenically cooled triple resonance $^{1}H$ [$^{13}C$/$^{15}N$] probe. All the solvents used were of the highest purity. Deuterated methanol ($CD_3OD$), deuterated DMSO (DMSO D6) and $^{13}C$ chloroform (*$CHCl_3$) were purchased from Eurisotop. Benzene-D6 was purchased from Sigma-Aldrich (Saint Quentin, France). Automated two-electrode voltage clamp TEVC was performed using a HiClamp device (MCS GmbH., Reutlingen, Germany). *Ingrilimine* and *portimine* were dissolved in ethanol and diluted in physiological solutions. The total ethanol concentration in the test solution did not exceed 0.15%.

### 1) Purification of compound (IIIa)

*Ingrilimine* was purified from an extract of *Vulcanodinium rugosum.* More particularly, *Ingrilimine* was purified from an ethanolic extract *of Vulcanodinium rugosum.*

All glassware was cleaned with acetone, rinsed with deionized $H_2O$, and dried in an oven at approximately 65 °C for several hours. An ethanolic extract (100 mL) was prepared by sonication from 1 g of lyophilized *Vulcanodinium rugosum.* The extract was diluted 10-times with $H_2O$ and aliquots of 100 mL were loaded by gravity onto 5g C18 SPE columns (Waters Sep-Pak, Waters) previously conditioned with 60 mL MeOH and 100 mL $H_2O$. The SPE columns were successively washed with 60 mL each of $H_2O$, 10% MeOH, 25% MeOH and 100% MeOH. The SPE fractions were screened for the presence of nAChR-ligands using a microplate receptor-ligand binding assay (Aráoz et al. (2012) Coupling the Torpedo Microplate-Receptor Binding Assay with Mass Spectrometry to Detect Cyclic Imine Neurotoxins. Analytical Chemistry 2012, 84(23):10445-10453 [23]). Pinnatoxin-G, *portimine-A,* and a novel nAChR-ligand of 370 *m/z* $[M+H]^+$, later named *ingrilimine,* were detected by liquid chromatography coupled to mass spectrometry (LC-MS) in the ligand binding positive fractions. The latter fractions were pooled and evaporated. The whole dried material (146.1 mg)

was resuspended in 1 mL DMSO. For HPLC purification, the elution profile of *ingrilimine* was optimized using an XBridge BEH 300 C18 column (5 μm, 4.6 × 250 mm, Waters). Purification of *ingrilimine* was performed using an XBridge C18 column (5 μm, 19 × 150 mm, Waters) monitored by a preparative HPLC system and recorded at 214, 230 and 254 nm, at a flowrate of 20 mL/min at room temperature. Solvent A was $H_2O$, 0.1% formic acid, and solvent B was $CH_3CN$, 0.1% formic acid. Forty mg of sample (300 μL in DMSO) was loaded onto the column. The elution profile was: isocratic 10% B, 0-5 min; isocratic 20% B, 5.1-15 min; isocratic 25% B, 15.1-20 min; isocratic 30% B, 20.1-25 min; gradient 30-100% B, 25.1-30 min; isocratic 100% B, 30.1-35 min; isocratic 0% B, 35.1-40 min. Fractions of 10 mL were collected and analyzed by HRMS using a QExactive Focus Orbitrap LC-MS/MS system (Thermo Scientific®). Under these conditions, portimine eluted as a broad major peak (retention time: 7.5-10 min), while *ingrilimine* eluted as a minor peak (retention time: 11.25-12.5 min). Portimine and *ingrilimine* fractions were pooled and dried.

From a 1.46 g extract (dry weight), 12.5 mg of *portimine-A* (0.86%) and 250 μg of *ingrilimine* (0.02%) were purified to homogeneity. *Ingrilimine* yielded a yellow powder with a maximal absorption $\lambda_{max}$ at 229.7 nm in methanol. IR (ATR, ṽ) = 3390.63, 2256.85, 2129.93, 1654.20, 1048.20, 1023.31, 993.53, 824.88, 762.50 $cm^{-1}$. HRMS of *ingrilimine* gave a molecular ion of *m/z* 370.2736 $[M+H]^+$, and a M, of 369.2663 with a chemical formula of $C_{24}H_{35}O_2N$. *Ingrilimine* is the smallest congener of this class of neurotoxins described to date.

**2) <u>Experimental details concerning NMR characterization of the compound (IIIa)</u>**

NMR experiments were recorded on either an 800 MHz Avance NEO (Bruker® Biospin, Billerica, MA, USA) spectrometer with an 18.1 tesla magnetic field or a 600 MHz Avance III HD spectrometer with a 14.1 tesla magnetic field for proton frequency, both equipped with a cryogenically cooled triple resonance $^1H[^{13}C/^{15}N]$ probe. Spectra were recorded using TOPSPIN 4.05 (Bruker® Biospin). The purified *ingrilimine* was dissolved in 290 μL of deuterated methanol ($CD_3OD$) and introduced in a 4 mm Shigemi tube (Shigemi, Alison Park, PA, USA) yielding a ~3 mM sample. Experiments for proton were run at 298 K, or 303 K with either a 5400 Hz or 7150 Hz sweep width for the 600 MHz and the 800 MHz, respectively. Spectra were referenced to external TMS (tetramethylsilane). The 2D proton NMR spectra (COSY, TOCSY, ROESY and NOESY) were collected with 2 K data points in the t2 dimension and 256 or 512 t1 increments, with typically 32 to 64 scans per increment. Two dimensional heteronuclear experiments were run at 303K on the 800 MHz, namely $^{13}C$ HSQC edit, $^{13}C$ HSQCTOCSY, $^{13}C$ HMBC and H2BC (Nyberg et al. (2005) Heteronuclear Two-Bond Correlation: Suppressing Heteronuclear Three-bond or higher NMR correlations while enhancing two-bond correlations even for vanishing 2JCH. Journal of the American Chemical Society, 127(17):6154-6155 **[24]**). DEPT135 and DEPTq were run on the 600 MHz spectrometer. Water suppression was achieved either by pre-saturation during the recycle delay or, in the NOESY experiment, by double pulsed-field gradient spin echoes (DPFGSE).

**Analysis of the Chemical Structure in $CD_3OD$:**

The molecular formula of the isolated compound was established as $C_{24}H_{35}O_2N$ (MW, 369.2663) from HRMS analysis leading to eight degrees of unsaturation.

The $^{13}C$ HSQC edited NMR spectrum shows 8 methine, 2 methyl and 9 methylene groups while the $^{13}C$ HMBC spectrum gives four additional quaternary carbons. The detailed analysis of both 1D proton and $^{13}C$ HSQC edited NMR spectra show characteristic features in terms of chemical shifts and proton J couplings, of a terminal diene $H_{21}$ and $H_{22a}H_{22b}$ ($\delta_H$ 6.40 ppm, dd, J = 17.5, 10.4 Hz ; 4.97 ppm, d, J = 10.5 Hz and 5.10 ppm, d, J = 17.5 Hz with $\delta_C$ 139.2 ppm, 109.8 ppm respectively for $C_{21}$ and $C_{22}$). Three additional double bonds can be inferred from the $^1H$ and $^{13}C$ chemical shifts ($\delta_H$ 5.77 ppm, 5.53 ppm, b; 5.09 ppm, b; and $\delta_C$ 129.04 ppm, 125.75 ppm, 124.3 ppm respectively). Two tertiary methyl groups ($\delta_H$ 1.68 ppm: 1.71 ppm and $\delta_C$ 14.3ppm; 17.3 ppm respectively), two oxymethine groups ($\delta_H$ 4.11 ppm; 3.89 ppm and $\delta_C$ 71.06 ppm; 78.32 ppm respectively) and nine methylene groups. This agrees with the DEPT135 experiment which shows the presence of 9 methylene carbons including one olefinic, eight methine carbons among which two oxygenated carbons and four olefinic as well as two methyl groups, while the DEPTq shows the presence of three additional quaternary carbons (135.5 ppm, 134.4 ppm and 134.4 ppm). The $C_4$ quaternary carbon (43,7 ppm) could not be observed as it is close the large signal of the methanol (47 ppm). However, the $^1H$-$^{13}C$ HMBC experiments allowed to identify 4 quaternary carbons ($\delta_C$ 43.7 ppm, 134.4 ppm, 135.5 ppm and 134.1 ppm). Thus, including the imine double bond there are three rings in the molecule (rings **A, B** and **C**) out of the eight degrees of unsaturation. All together 23 carbons signals are identified while one quaternary carbon could not be depicted.

Then combination of heteronuclear $^{13}C$-$^1H$ HSQC, H2BC, HSQCTOCSY and HMBC 2D experiments as well as homonuclear COSY and TOCSY 2D experiments allow to establish the structure of this molecule. The 2D proton experiments COSY and TOCSY allow partial assignments of the three ring structures, that are then connected *via* the HMBC experiment. First for ring **A,** the downfield-shifted methylene group ($\delta_H$ 3.53 ppm $H_{1a}$ $H_{1b;}$ dc 48.24 ppm, $C_1$) likely to be close to the imine, by analogy with previously described cyclic spiroimine, is through bond connected to two methylene groups ($\delta_H$ 1.67 ppm, 1.61 ppm $H_{2a}H_{2b}$ and 1.74 ppm, 1.51 ppm, $H_{3a}H_{3b}$ suggesting that the imine ring is a six-member ring

(ring A). A heteronuclear multiple bond (HMBC) correlation cross peaks is also observed from $H_2$ ($\delta_H$ 1.67 ppm) to the quaternary carbon $C_4$ ($\delta_C$ 43.7 ppm).

Then, the conjugated vinyl group $H_{22a}$ $H_{22b}$ ($\delta_H$ 4.97, 5.10; dc 109.8) and $H_{21}$ ($\delta_H$ 6.41, $\delta_C$ 139) is attached to a cyclohexene (ring C) as evidenced from both COSY correlations ($H_{21}/H_{17}/H_{16}$) and HMBC correlations ($H_{21}/C_{17}$, $H_{21}/C_{18}$ and $H_{21}/C_{19}$) but also COSY correlations ($H_{17}/H_{16}$ and $H_{17}/H_{19}$) as well as HMBC correlations ($H_{17}$ with $C_4$, $C_{15}$, $C_{16}$, $C_{19}$ and $C_{21}$). The multiple bond correlations observed with $C_4$, a quaternary carbon, allowed to attach the cyclohexene ring **C** to the ring **A.** In conclusion the cyclohexene of the 6,6, spirocyclic imine moiety of *ingrilimine* bares a diene as it is the case of kabirimine (Hermawan et al. (2019) Kabirimine, a new cyclic imine from an Okinawan dinoflagellate. Marine Drugs, 17(6) **[25]**). However, the quaternary carbon expected at position 5 was not observed.

Finally, starting from $H_{16}$ ($\delta_H$ 3.34 ppm) a proton from the cyclohexene moiety combining COSY TOCSY and HMBC it was possible to identify all protons and carbons of segment 16 to 7. Finally, in the COSY spectrum a strong interaction is observed between the two methylene protons $H_{7a}$ and $H_{7b}$ (2.783 ppm, 2.12 ppm) but also from $H_{7a}$ (2.783 ppm) with a proton resonating at 2.85 ppm that must correspond to $H_6$. In the HSQC edited spectrum this proton is carried by a carbon resonating at $\delta_C$ 30.66 ppm and corresponds to a methine group. Thus, from carbon 6 to carbon 16 we have a structure with 11 carbons corresponding to ring **B.** A weak through bond connection observed in the TOCSY spectrum between $H_{1a1b}$ with the He proton resonating at 2.85 ppm but also with the $H_{7a}$ proton resonating at 2.78 ppm allows to attach ring **B** to ring **A.** In addition, the H2BC shows that the $H_6$ proton at 2.85ppm is linked to the carbon $C_7$ (22.7ppm) and is spatially close to the $H_{16}$ proton as shown in the NOESY spectrum. NMR recordings were performed several times under different conditions (pH, EDTA addition) and large variations in proton chemical shifts for $H_6$, $H_{15a}H_{15b}$ and $H_{16}$ and to a slighter extent for $H_{19a}$ and $H_{19b}$ were observed. All these protons are close to the imine functional group of the 6,6, spiroimine moitety. At this stage in the deuterated methanol solvent the molecular formula established from these results is $C_{23}H_{34}O_2N$ corresponding to a molecular weight of 356, therefore one CH is missing. All proton and carbon chemical shifts have been conforted with the HSQC-TOCSY experiment (Table 2).

**Table 2.** NMR data of ingrilimine (800 MHz, in $CD_3OD$).

| Position | δC | δH (mult; J Hz) | J coupling |
|---|---|---|---|
| 1 | 48.24[a] | 3.536[a] | |
| | | 3.536[b] | |
| | 19.05[a] | 1.677[a] | |
| | | 1.607[b] | |
| 3 | 23.86[a] | 1.747[a] | |
| | | 1.571[b] | |
| 4 | 43.7[a] | | |
| 5 | | | |
| 6 | 30.66 | 2.853 | 7H[a] 6H: 12.5 Hz |
| 7 | 22.7[a] | 2.783[a] | 7H[a] 8H: 8.8Hz |
| | | 2.121[b] | |
| 8 | 124.35 | 5.0978 | |
| 9 | 134.14 | | |
| 10 | 34.86[a] | 2.826[a] | 10H[a] 11H: 7Hz |
| | | 2.529[b] | 10H[b] 11H: 7Hz |
| 11 | 125.75 | 5.536 | |
| 12 | 135.5 | | |
| 13 | 78.42 | 3.895 | 13H 14H: 2Hz |
| 14 | 71.26 | 4.109 | 14H 15H[a]: 11.4Hz |
| | | | 14H 15H[b]: 3.4Hz |
| 15 | 32.59[a] | 1.429[a] | 15H[a] 16H: <3Hz |
| | | 1.23[b] | 15H[b] 16H: 12.Hz |
| 16 | 36.73 | 3.342 | |
| 17 | 129.04 | 5.769 | 17H 16H: < 1Hz |
| 18 | 134.4 | | |
| 19 | 20.42[a] | 2.306[a] | |
| | | 2.208[b] | |
| 20 | 31.7[a] | 1.967[a] | |

(continued)

| Position | δC | δH (mult; J Hz) | J coupling |
|---|---|---|---|
| | | 1.666[b] | |
| 21 | 139 | 6.401 | |
| 22 | 109.78[a] | 5.112[a] | 22H[a] 21H: 17.5Hz |
| | | 4.968[b] | 22H[b] 21H: 10.84Hz |
| 23 | 14.3 | 1.682 | |
| 24 | 17.19 | 1.713 | |

[a] Downfield signal; [b] upfield signal.

**Analysis of the Chemical Structure in DMSO D6:**

The proton spectrum obtained is similar to that obtained in $CD_3OD$. Two new signals are observed at 4.76 ppm and 4.379 ppm that correspond to the two hydroxyl groups.

Interestingly the $^{13}C$ HSCQ NMR spectrum shows 7 methine, 2 methyl and 10 methylene groups while the $^{13}C$ HMBC spectrum gives also four additional quaternary carbons. Thus, the methine group at position 6 observed in methanol solvent ($\delta_H$ 2.85 ppm, $\delta_C$ 30.66 ppm) appears here as a methylene group ($\delta_H$ 2.42 ppm and 1.99 ppm, $\delta_C$ 30.96 ppm). Similarly, to that observed in the methanol solvent, in the TOCSY experiment an homoallylic coupling is observed between $H_{1a}H_{1b}$ and both $H_{6a}H_{6b}$ and $H_{7a}H_{7b}$. Added to this, the two hydroxyl group positions 13 and 14 of the two oxymethine groups could be identified at position 4.75 ppm and 4.375 ppm respectively. Similarly, to the analysis achieved in deuterated methanol the $C_5$ carbon could not be detected. However, as also observed in $CD_3OD$, there is a strong dipolar interaction between the proton $H_6$, ($\delta_H$ 2.42 ppm) and $H_{16}$ ($\delta_H$ 3.1 ppm) observed both in the NOESY and the ROESY spectra as well as in the selective 1D NOE experiments. As in the case of NMR recordings of *ingrilimine* in $CD_3OD$, the quaternary carbon at position 5 could not be depicted in $DMSOd_6$.

To overcome the problem of the missing carbon the dimethyl sulfoxide sample was slightly acidified, and proton and carbon resonance assignments repeated. Assignments in more acidic conditions were quite straightforward although substantial variations in chemical shifts could be also observed. This is particularly true for the protons and the carbons of ring **A** but also the methylene 6 (shift over 0.6 ppm) as well as methylene 15 and methine 16 of ring **C.** Moreover, after a year this methylene group becomes a methine group resonating at 3 ppm while a weak population of the methylene is remaining suggesting a slow conformational equilibrium. Although the carbon 5 wasn't identified in this solvent it was still possible to attach ring **B** to ring **A** *via* the correlation observed in a 1D selective TOCSY when irradiating protons 1a and 1b at 3.43 ppm and 3.34 ppm respectively of ring **A** interaction with the methylene protons 6 and 7 are observed. All proton and carbon chemical shifts have been conforted with the HSQC-TOCSY experiment (Table 3).

The missing quaternary carbon at position 5 together with the fact that a methine or a methylene group is identified at position six depending on the solvent but also on pH conditions and their proximity to the imine would suggest, as already pointed out in the case of gymnodimine, an imine-enamine tautomerism (Seki et al. (1995) Gymnodimine, a new marine toxin of unprecedented structure isolated from New-Zealand oysters and the dinoflagellate, Gymnodinium sp. Tetrahedron Letters, 36(39):7093-7096 **[26]**). This phenomenon has been observed for other related CiTXs like pinnatoxin H (Selwood et al. (2014) Pinnatoxin H: a new pinnatoxin analogue from a South China Sea Vulcanodinium rugosum isolate. Tetrahedron Letters, 55(40):5508-5510 **[27]**).

**Table 3.** NMR data of ingrilimine (800 MHz, in DMSO D6).

| Position | δC | δH (mult; J Hz) |
|---|---|---|
| 1 | 49.6[a] | 3.439[a] |
| | | 3.363[b] |
| 2 | 20.10[a] | 1.479[a] |
| | | 1.395[b] |
| 3 | 24.88[a] | 1.466[a] |
| | | 1.365[b] |
| 4 | 41.98 | |
| 5 | | |
| 6 | 30.96[a] | 2.42[a] |
| | | 1.999[b] |
| 7 | 22.96[a] | 2.718[a] |

(continued)

| Position | δC | δH (mult; J Hz) |
|---|---|---|
| | | 1.802[b] |
| 8 | 126.88 | 5.025 |
| 9 | 132.89 | |
| 10 | 35.3[a] | 2.734[a] |
| | | 2.345[b] |
| 11 | 125.25 | 5.328 |
| 12 | 136.31 | |
| 13 | 78.6 | 3.701 |
| | | OH 4.76 |
| 14 | 71.7 | 3.904 |
| | | OH 4.37 |
| 15 | 31.94[a] | 1.305[a] |
| | | 1.000[b] |
| 16 | 37 | 3.098 |
| 17 | 131.86 | 5.701 |
| 18 | 134.17 | |
| 19 | 21.02[a] | 2.152[a] |
| | | 2.072[b] |
| 20 | 31.35[a] | 1.75[a] |
| | | 1.526[b] |
| 21 | 139.82 | 6.341 |
| 22 | 111.04[a] | 5.054[a] |
| | | 4.918[b] |

[a] Downfield signal; [b] upfield signal.

**Structural features of *ingrilimine.***

The conformation around the 5 double bonds was inferred for three of them $C_8$-$C_9$, $C_{11}$-$C_{12}$ and $C_{21}$-$C_{22}$, from analysis of $^3$J-coupling and dipolar interactions both in methanol and in dimethyl sulfoxide, the two remaining ones the imine and the $C_{17}$-$C_{18}$ being already constraint. Thus, for the $C_8$ $C_9$ bond, although the proton 8 is quite broad it appears as a doublet with a coupling constant of about 8.8 Hz. Selective irradiation of proton $H_{7a}$ (2.78 ppm) suppresses the 8.8 Hz coupling constant while irradiation of $H_{7b}$ (2.13 ppm) did not perturb the $H_8$. The lack of strong dipolar interaction between the $H_8$ proton at 5.10 ppm with the methyl group 24 (1.71 ppm) is in favor of a *trans* conformation for these two groups leading to an E conformation for this bond. For the $C_{11}$ $C_{12}$ double bond, the $H_{11}$ proton resonating at 5.53 ppm is also broad and display a triplet structure implying that it has similar coupling constants with the two protons $H_{10}$. Proton $H_{11}$ displays a strong dipolar interaction with proton $H_{12}$ (3.90 ppm) and the two $H_{10}$ protons (2.82 ppm and 2.53 ppm) but no interaction with the methyl group 23 (1.68 ppm). The proton $H_{11}$ (5.53 ppm) is in position *trans* with the methyl group 24 leading to an E conformation for this bond. Finally, double bond $C_{21}$ $C_{22}$ is compatible with a *trans* conformation of proton $H_{21}$ with proton $H_{22a}$ this in agreement firstly with the three bond coupling constants $^3J_{H21H22a}$ of 17.69 Hz and $^3J_{H21H22b}$ of 10.68 Hz but also with the strong dipolar interactions observed between $H_{21}$ and $H_{22}$b as well as between $H_{22a}$ and the two protons $H_{19a}$ and $H_{19b}$. The NOE observed between $H_{21}$, $H_{17}$ and $H_{16}$ together with the very small coupling constant $^3J_{H16H17}$ of nearly 0Hz fixes the conformation around the double bond $C_{17}$ $C_{18}$. These results are attested in the dimethyl sulfoxide solvent.

They are four asymmetric carbons $C_4$, $C_{13}$, $C_{14}$ and $C_{16}$. Selective decoupling of protons $H_{16}$ and $H_{14}$ give access to the three bond coupling constants between protons $H_{16}$ with the two protons $H_{15}$ and between these two protons and proton $H_{14}$. Added to this, proton $H_{13}$ which is only coupled to $H_{14}$ displays a small coupling of about 2 Hz while the lack of dipolar interaction between the two hydroxyl groups 13 and 14 would suggest that stereochemistry of carbons 13 is S and that of 4 and 14 is R. Dipolar interactions between the protons, $H_7$, $H_8$, $H_{10b}$, $H_{12}$, $H_{15a}$, $H_{16}$ were observed, and the substituents priority $C_5 > C_{16} > C_3 > C_{20}$ are in favor of a S stereochemistry for $C_4$.

**Part II. biochemical study and activity of the molecules (Methods)**

**1. Toxin quantification.** Because *ingrilimine* is a novel CiTX and of the lack of certified standards for portimine, the quantification of both toxins was done by NMR spectroscopy. The same stock of both toxins was used for the whole biological experiments. $^1H$ NMR Quantification of *ingrilimine* and portimine was performed as described by Profesor Armen Zakarian (Araoz et al., 2011). Briefly, a stock solution of chloroform ($CHCl_3$) and benzene (PhH) was prepared by pipeting 7.0 μL $CHCl_3$ (10.44 mg; 0.08749 mmol) and 7.0 μL PhH (6.12 mg; 0.7833 mmol) into 7.0 mL of $CD_3OD$ (Euriso-Top). The concentration of the internal samples in this solution was 0.012489 M for $CHCl_3$, and 0.011189 M for PhH. After mixing, 0.5 mL of this solution was used to dissolve the dried *ingrilimine* or *portimine* samples to be quantified in a 2 mL glass bottle. The whole was added to an NMR tube and a $^1H$-NMR was recorded as described above.

After phasing the spectrum and performing baseline corrections, $CHCl_3$, PhH, and several protons in the range of -0.9 ppm to 6.0 ppm were integrated. The quantification of the toxins was done according to the formula considering 1.00 mL final volume mix:

$$(1)\ M\ sample = (integral\ sample/\ integral\ CHCl_3) \times 0.012489\ M\ CHCl_3$$

$$(2)\ M\ sample = (integral\ sample/\ integral\ PhH) \times 0.011189\ M\ PhH$$

These calculations were repeated with several peaks throughout the spectrum of the sample and an average of the molarity of the sample was taken.

**2. Cell Culture and Proliferation Assay.** Cancer cell lines were obtained from the American type Culture Collection (Rockville, MD) and cultured according to the supplier's instructions. A2780 ovarian and HT29 colorectal carcinoma cells were grown in RPMI 1640 supplemented with 10% fetal calf serum (FCS) and 1% glutamine. U87-MG glioblastoma cells were grown in Dulbecco minimal essential medium (DMEM) containing 4.5 g/L glucose, 10% FCS and 1% glutamine. All cell lines were maintained at 37 °C in a humidified atmosphere containing 5% $CO_2$.

For $IC_{50}$ determination, the cells were seeded in 96-well plates ($3 \times 10^3$ cells/well) containing 90 μL of growth medium. After 24 h of culture, the cells were treated with the toxin compounds at 10 different final concentrations. Each concentration was obtained from serial dilutions in culture medium starting from the stock solution. Control cells were treated with the vehicle (DMSO). Experiments were performed in triplicate.

After 72 h incubation, 100 μL of CellTiter Glo Reagent (Promega, Madison, WI, USA). was added for 15 min before luminescence recording with a spectrophotometric plate reader PolarStar Omega (BMG LabTech). The cell viability data of treated cells were normalized to the viability values of DMSO-treated cells. Dose-response curves and the $IC_{50}$ values were calculated using the Graph Prism software

**3. Apoptosis induction by *ingrilimine.***

**Measurement of mitochondrial membrane potential.** One of the hallmarks for apoptosis is the loss of mitochondrial membrane potential ($\Delta\Psi m$). The changes in the mitochondrial potential were detected by using 5,5',6,6'-tetrachloro-1,1',3,3' tetraethylbenzimidazolylcarbocyanine iodide/chloride (JC-1), a cationic dye that exhibits potential dependent accumulation in mitochondria, indicated by the fluorescence emission shift from red (590 nm) to green (525 nm). In brief, HCT116 cells were treated with *ingrilimine* in the range of 10-500 nm for 24h and 48h. After treatment, cells were resuspended in 1 mL of PBS containing 2 μM of JC-1 probe (final concentration) and incubated at RT for 15 min. Analysis of cells was performed on a FC500 flow cytometer (Beckman Coulter®, France).

**Measurement of phosphatidylserine exposure on the outer plasma membrane.** The apoptosis of *ingrilimine-treated* A2780 cells was also assessed by flow cytometry using propidium iodide and an FITC Annexin V apoptosis detection kit (BD Biosciences, San Jose, CA, USA). FITC-conjugated annexin V has been used to detect the externalization of phosphatidylserine that occurs at an early stage of apoptosis. Propidium iodide is a marker for necrosis due to cell membrane destruction.

4. Two-electrode voltage clamp electrophysiology.

Adult *Xenopus laevis* animals were from TEFOR Paris-Saclay (Gif sur Yvette, France). Animal housing, handling and experimental procedures were carried according to the Ethical Committee for Animal Experimentation C2EA-59 of Paris Center-South. Oocyte extraction from anesthetized female frogs was performed by laparotomy. The follicular layer of stage V-VI oocytes were removed with tweezers under a binocular microscope in Barth's solution (88 mM NaCl, 1 mM KCl, 0.41 mM $CaCl_2$, 0.82 mM $MgSO_4$, 2.5 mM NaHCOs, 0.33 mM $Ca(NO_3)_2$, 7.5 mM Hepes; pH 7.6) supplemented with 0.1 mg/mL kanamycin. *X. laevis* oocytes were nanoinjected with 50 nL of mRNA (1 μg/μL) coding for the α7 nAChR subunit, or

a with 50 nL of a 1:1 mix of α3 and β2 mRNA (1 μg/μL), or with 50 nL of purified *Torpedo marmorata* electrocyte membranes (2.5 μg/μL protein) rich in $(\alpha 1)_2\beta\gamma\delta$ muscle nAChR (muscle nicotinic acetylcholine receptors). The oocytes were incubated in Barth's solution at 18 °C.

Automated TEVC was performed using a HiClamp device (MCS GmbH., Reutlingen, Germany) and OR2 as buffer solution (88 mM NaCl, 1mM $MgCl_2$, 5mM Hepes; pH 7.6). A single oocyte is withdrawn from a 96-wells oocyte-microplate and placed in a silver wire basket that integrates a reference bath electrode. The oocyte is automatically impaled with two borosilicate glass microelectrodes filled with a mix of 1M KCl and 1.5 M KAc (0.5-5 MΩ resistance) and clamped at a holding potential of -60 mV. For TEVC recording, the clamped oocyte was incubated in a well of the sample-microplate containing OR2 for 45 s; then it was transferred into a second well containing 100 μM ACh for 5 s, after what it was placed in the washing station and perfused with OR2 for 4 min. ACh-evoked control currents were recorded thrice (three times) as being described above to obtain the $I_{ACh}$ peak amplitude. The same clamped oocyte was incubated with *ingrilimine* at a given concentration for 45 s, immediately after, it was transferred for 5 s into a second well containing a mix of 100 μM ACh and *ingrilimine* at the same given concentration. The clamped oocyte was transferred to the washing station for 4 min. After testing the effect of *ingrilimine* the same clamped oocyte is incubated with 100 μM ACh under control conditions as describe above.

Agonist dose-response curves were obtained using the equation:

$$(3):\ I = I_{max}\ [L]^n / (EC_{50} + [L])^n$$

where, $I$ is the agonist evoked current, $[L]$ is the agonist concentration, $EC_{50}$ is the agonist concentration that evokes 50% of the maximal current recorded ($I_{max}$), and $n$ is the Hill coefficient.

Antagonist dose-response inhibition curves were obtained using the equation:

$$(4):\ Y = 100 / (1+10^{((\mathrm{Log}\ IC_{50} - [X]) \times n))})$$

where, $Y$ is the fractional remaining response in percentage (%), in the presence of a given concentration of antagonist $[X]$, $IC_{50}$ is the antagonist concentration that reduced by 50% the ACh-evoked amplitude, and $n$ is the Hill coefficient. Concentration-response curves were obtained by incubating a *Xenopus* oocyte expressing a given nAChR with *ingrilimine* or *portimine* in the range of 1 pM - 10 μM concentration.

**Results:**

**Cell viability.** *Ingrilimine* is a potent cytotoxic compound against the three cancer cell lines tested (A2780, HT29 and U87-MG, Figure 2) with $IC_{50}$ (half-maximal inhibitory concentration) values ranging from 10-45 nM. The cell viability assay was performed using *portimine*-A as a positive control (Table 4). Although *ingrilimine* displayed a strong cytotoxic activity in the nanomolar range, it was 13-times less potent than *portimine*-A to kill the A2780 ovarian cancer cells, while its cytotoxic activity against the HT29 and the U87-MG cancer cells lines was 5-times less potent compared to *portimine*-A (Table 4). The $IC_{50}$ data obtained for *portimine*-A correlate with the antiproliferation activity of *portimine*-A obtained against a panel of 20 human and mouse cancer cell lines (Tang et al (2023) synthesis of portimines reveals the basis of their anti-cancer activity. Nature, 622(7983): 507-513 **[28]**). Aside *portimine* A, *ingrilimine* and *portimine* B that showed cytotoxic activities in the nanomolar range ($10^{-9}$ M, $10^{-8}$ M and $10^{-7}$ M, respectively) (For references: Tang et al. (2023) **[28],** this work for *ingrilimine,* and Fribley et al. (2019) Identification of portimine B, a new cell permeable spiroimine that induces apoptosis in oral squamous cell carcinoma. ACS Medicinal Chemistry Letters, 10(2): 175-179 **[29]),** other CiTX like the extremely lethal to mice pinnatoxin-F is less cytotoxic against the mouse P338 leukemia cell line with an $IC_{50}$ > 1 μM (Hampton et al. (2014) **[22]**). Gymnodimine was also cytotoxic, but in the micromolar region against the normal cell lines 3T3 and L929 and the tumor cell line 4T1 from mice and against the human intestinal epithelial Caco-2 cells (Wang et al. (2023) Apoptosis and oxidative stress of mouse breast carcinoma 4T1 and human intestinal epithelial Caco-2 cell lines caused by the phycotoxin gymnodimine-A. Chemico-Biological Interactions, 384 **[30]**).

**Table 4.** Cytotoxic activity of *portimine-A* and *ingrilimine*

| CiTX | $IC_{50}$ ± SEM[a] (nM) | | |
|---|---|---|---|
| | A2780 | HT29 | U87-MG |
| *Portimine*-A | 0.77 ± 0.1 | 6.40 ± 0.1 | 8.10 ± 7.8 |
| *Ingrilimine* | 10.6 ± 0.2 | 34.3 ± 1.1 | 44.2 ± 0.3 |

[a] Mean value ± SEM from three distinct experiments performed in duplicate.

**Apoptosis:**

**Mitochondrial membrane potential (JC-1).** Mitochondria are essential for life (bioenergetics, respiratory chain). Disruption of mitochondria is considered to occur at early stages of apoptosis. The mitochondrial mediated caspase activation is a major apoptotic pathway characterized by mitochondrial outer membrane permeabilization. This is preceded by the loss of membrane potential that is considered as an early event of apoptosis in some apoptotic systems (Ly et al. (2003) The mitochondrial membrane potential ($\Delta\psi$m) in apoptosis; an update. Apoptosis, 8(2):115-128 **[31].** Mitochondrial membrane potential is a primary indicator of mitochondrial function. The electrochemical membrane potential across the internal mitochondrial membrane has been estimated to be -180 mV for isolated respiring mitochondria, however this value can vary among cell types displaying significant intracellular heterogeneity (Harrington et al. (2023) Mitochondria in health, disease, and aging. Physiological Reviews 2023, 103(4):2349-2422 **[32]**). To assess the effects of *ingrilimine* on the mitochondrial membrane potential by flow cytometry, JC-1, a potential-dependent fluorescent dye indicator that accumulates in mitochondria has been used. JC-1 dye is widely used in apoptosis studies to monitor mitochondrial health: a fluorescence emission shift from red ($\lambda$ 590 nm) to green ($\lambda$ 529 nm) denotes mitochondrial membrane potential depolarization. As shown in Figure 3a, non-treated cells incubated for 24 h emit fluorescence at 590 nM due to the accumulation of JC-1 in their mitochondria (sector Q). Following 24 h incubation of human colorectal carcinoma HCT116 cells with 10 nM *ingrilimine,* 5.1% of the treated cells showed a change in their fluorescence emission from 590 nm to 529 nm (Figure 3b; sector Q). When HCT116 were treated with 250 nM *ingrilimine* for 24 h the percentage of HCT116 cells showing membrane depolarization increased to 22.7% (Figure 3c; sector Q). The apoptotic cells increased with the incubation time. Thus, after 48h, 64.1% of the HT116 cells treated with 250 nM *ingrilimine* underwent apoptosis (Figure 3f; sector Q). No major variation in fluorescence shift were recorded in non-treated cells, and those treated with 10 nM *ingrilimine* after 48 h incubation (Figure 3a and 3b, 3e; sector Q).

HCT116 cells were also treated with *portimine,* used here as a positive control. As shown in Table 5. *Portimine* was 10-times more potent than *ingrilimine* in inducing mitochondrial membrane depolarization: 31.6% of the HCT116 cells treated with 5 nM *portimine* underwent apoptosis. The highest value recorded was 62.3% of apoptotic cells after 48 h treatment with 20 nM *portimine.* This agrees with previous studies that showed that *portimine* induces early stages of apoptosis as evidenced by caspase-3 activation (Tang et al. (2023) **[28]**). In conclusion, *ingrilimine* can induce apoptosis by mitochondrial membrane depolarization path, albeit it is 10-times less potent than *portimine.*

**Table 5.** Apoptotic activity of *portimine-A* and *ingrilimine.* JC-1 Mithocondrial membrane potential assay

| | JC-1 % | |
|---|---|---|
| | 24 h | 48 h |
| NT | 5.3 | 3.3 |
| *Portimine* [nM] | | |
| 1 | 5.1 | 8.1 |
| 5 | 9.2 | 31.6 |
| 10 | 19.7 | 44.6 |
| 20 | 23.9 | 62.1 |
| 50 | 37.0 | n.d. |
| *Ingrilimine* [nM] | | |
| 10 | 5.1 | 4.4 |
| 50 | 6.1 | 22.6 |
| 100 | 14.9 | 44.1 |
| 250 | 22.7 | 64.1 |
| 500 | 36.9 | n.d. |

**Phosphatidylserine exposure.** The induction of apoptosis following incubation of A2780 ovarian carcinoma cells with different concentrations of *ingrilimine,* and *portimine* as positive control, was quantified by flow cytometry using FITC Annexin V apoptosis kit that detects phosphatidylserine exposure. In early apoptotic cells, phosphatidylserine is translocated from the inner membrane leaflet facing the protoplasm of the cell to the outer leaflet facing the extracellular space. Annexin V, even conjugated with the fluorophore FITC, retained its high affinity for binding phosphatidylserine and it is used for detecting apoptosis at an early stage. However, as phosphatidylserine may be also exposed at latest stages of cell death, the staining with FITC Annexin V was performed (according to the manufacturer's instructions) in conjunction with the vital dye propidium iodide. Early stage apoptotic cells exclude the vital dye and are therefore, propidium iodide

negative and annexin V positive.

Control cells incubated with DMSO are propidium iodide negative and annexin V negative (Figure 4a, d; quadrant D3). Incubation of A2780 cancer cells with 10 nM *ingrilimine* does not trigger phosphatidylserine exposure (Figure 4b and 4e). Incubation of A2780 cells with 250 nM *ingrilimine* triggered phosphatidylserine exposure in -13% of the cells following incubation for 24 h. Early stage apoptotic cells exclude the vital dye and are therefore, propidium iodide negative and annexin V positive (Figure 4c, quadrant D4). Following a 48-h treatment with 250 nM *ingrilimine* the proportion of early apoptotic cells increased to 31.3% (Figure 4f, quadrant D4). At the same time, the percentage of propidium iodide positive and annexin V positive increased from 3.8% to 17% in the same lapse of time (Figure 4c et 4f; quadrant D2: late stage apoptotic, already dead cells or necrotic cells). *Portimine* at a concentration of 20 nM was able to induce phosphatidylserine exposure in 13% of the A2780 ovarian carcinoma cells after 24 h treatment and 39% propidium iodide negative/ annexin V positive cells after 48 h. In a similar way than *ingrilimine,* the percentage of late-stage apoptotic cells and or necrotic cells increased from 4.3% to 15% in the same lapse of time (Table 6). *Ingrilimine* is -10-times less potent as early-stage apoptosis inducer than *portimine.* It is worth noticing that regardless of the high toxin concentrations and the prolonged treatment time, both toxins do not show cytotoxicity on the apoptosis-resistant ovarian carcinoma cells A2780 with 45 and 40% viable cells after 48 h treatment with 250 nM *ingrilimine* and 50 nM, respectively (Figure 4a, 4d, quadrant D3 and Table 6). The latter is consistent with the proportion of early-stage apoptotic Jurkat cells following incubation with 50 nM *portimine* for 6 h that yielded 50% annexin V-positive cells and 50% of viable cells (Hampton et al. (2014) **[22]).**

***Ingrilimine* activity on nAChRs.** Bio-guided purification of *ingrilimine* early denoted that it was active on nAChRs. Thus, functional studies of purified *ingrilimine* using two-electrodes voltage clamp (TEVC) electrophysiology on *Xenopus laevis* oocytes expressing the human $\alpha$7 nAChR confirmed the high affinity of this toxin for this receptor. While performing a dose-response curve in the range of 1 pM to 10 $\mu$M of *ingrilimine,* it was noticed that *ingrilimine* displayed a dual activity of towards $\alpha$7 nAChR: *i*) at nanomolar concentrations, *ingrilimine* fully antagonized the $\alpha$7 nAChR subtype in a concentration dependent manner with an $IC_{50}$ of 2.29 nM (Table 1, Figure 5), and *ii*) at micromolar concentrations, *ingrilimine* was able to open the receptor's channel (agonist activity) (Figure 6). The TEVC recording protocol considered the evaluation of agonist and antagonist behavior of a given molecule in the same experiment: an oocyte expressing the $\alpha$7 nAChR was first incubated with a given concentration of *ingrilimine* for 45 s and immediately after it was incubated in a mixture composed of 100 $\mu$M ACh and *ingrilimine* at the same given concentration for 5 s. When the oocyte expressing the $\alpha$7 nAChR was incubated with 1 $\mu$M *ingrilimine,* it immediately triggered the opening of the receptor's channel. The peak amplitude elicited by 10 $\mu$M *ingrilimine* was equivalent to -25% of the peak amplitude evoked by 100 $\mu$M ACh (Figure 6). Higher concentrations of *ingrilimine* could not be tested due to the reduced amount of purified material available (Figure 7).

**Table 6.** Apoptotic activity of *portimine-A* and *ingrilimine.* Phosphatidylserine assay (Annexin V staining)

| | Annexin V+/IP | |
|---|---|---|
| | 24 h | 48 h |
| NT | 2.8 | 0.1 |
| *Portimine* [nM] | | |
| 1 | 3.4 | 1.7 |
| 5 | 6.4 | 18.4 |
| 10 | 13.1 | 24.1 |
| 20 | 13.0 | 39.3 |
| 50 | 22.8 | n.d. |
| *Ingrilimine* [nM] | | |
| 10 | 1.1 | 0.8 |
| 50 | 3.4 | 10 |
| 100 | 9.8 | 20.4 |
| 250 | 13.1 | 31.3 |
| 500 | 27.1 | n.d. |

The cyclic imines studied to date behave as competitive antagonists of muscle and neuronal nAChR with high potency and broad subtype selectivity. The extent of the inhibition is dependent on the nAChR subtype and on the CiTx analyzed (Table 1). Thus, the 7,6 spiroimine-based pinnatoxin-A showed the best selectivity profile: the antagonistic activity of pinnatoxin-A against human $\alpha$7 nAChR was 50-times more potent than its antagonistic activity towards muscle-type $(\alpha1)_2\beta1\gamma\delta$ nAChR, and ~300-times more potent against the human neuronal $\alpha4\beta2$ nAChR subtype. In contrast, pinnatoxin-G that harbors a vinyl group at C-33 instead of a carboxylate like in pinnatoxin-A, has a lower affinity for the $\alpha$7

nAChR (Aráoz et al. (2011) **[5]**). Co-crystallization of pinnatoxin-A and pinnatoxin-G with the acetylcholine binding protein (AChBP) showed that the carboxylate group of pinnatoxin-A stablished hydrogen bonds with Ser165 of loop F and several water-mediated interactions with Tyr188 of loop C. In contrast, the vinyl group of pinnatoxin-G stablished only weak interactions with Ser165 (Bourne et al. (2015) Marine macrocyclic imines, pinnatoxins A and G: Structural determinants and functional properties to distinguish neuronal $\alpha$7 from muscle ($\alpha$1)2$\beta$1$\gamma\delta$ nAChRs. Structure 2015, 23(6): 1106-1115 **[33]**), explaining their differential antagonistic activities towards $\alpha$7 nAChR.

Along the same line, the 7,6 spiroimine-based 13-desmethyl spirolide-C, 13,19-didesmethyl spirolide-C and 20-methyl spirolide-G and the 6,6 spiroimine-based gymnodimine-A displayed sub nanomolar affinities against muscle and neuronal nAChR subtypes (Bourne et al. (2010) Structural determinants in phycotoxins and AChBP conferring high affinity binding and nicotinic AChR antagonism. Proceedings of the National Academy of Sciences of the United States of America, 107(13):6076-6081 **[34],** Aráoz et al. (2015) The neurotoxic effect of 13,19-didesmethyl and 13-desmethyl spirolide C phycotoxins is mainly mediated by nicotinic rather than muscarinic acetylcholine receptors. Toxicological Sciences, 147(1):156-167 **[35],** Couesnon et al. (2016) The dinoflagellate toxin 20-methyl spirolide-G potently blocks skeletal muscle and neuronal nicotinic acetylcholine receptors. Toxins, 8(9) **[36]**). However, prorocentrolide and portimine showed micromolar antagonistic activities towards muscle and neuronal nAChRs (Amar et al. (2018) Prorocentrolide-A from cultured Prorocentrum lima dinoflagellates collected in Japan blocks sub-types of nicotinic acetylcholine receptors. Toxins, 10(3) **[37],** Lamoise et al. (2017) **[3]**). Prorocentrolide harbors a planar hexahydroisoquinoline containing un imine group and a C27 macrolide both imbibed in a planar C49 macrocycle (Torigoe et al. (1988) **[2]**)**.** Docking calculations showed the absence of the hydrogen bond between the iminium group of prorocentrolide-A and the backbone carbonyl group of Trp147 in the acetylcholine-binding pocket that is characteristic for the interaction with other CiTXs (Couesnon et al. (2016) **[36]**). In the case of *portimine,* the 5,6 spiroimine may be responsible for the low affinity of *portimine*-A for muscle and neuronal nAChRs (Figure 5).

Thus, several lines of evidence pointed out the cyclic spiroimine as the pharmacophore of CiTXs essential for toxicity as well as for binding to nAChR. On the one hand, the hydrolysis of the 7,6 spiroimine of spirolide-A and spirolide-B that yielded the compounds spirolide-E and spirolide-F respectively, abolished their toxicity when tested by mouse bioassay (Hu et al. (1996) Characterization of biologically inactive spirolides E and F: Identification of the spirolide pharmacophore. Tetrahedron Letters 1996, 37(43):7671-7674 **[38]**). Similarly, the chemical hydrolysis of the 6,6 spiroimine of gymnodimine yielded a less toxic gymnodamine compound (Stewart et al. (1997) The absolute stereochemistry of the New Zealand shellfish toxin gymnodimine. Tetrahedron Letters, 38(27):4889-4890 **[39]**). The chemical synthesis of the amino keto pinnatoxin-A in which the 7,6 spiroimine of pinnatoxin-A was open confirmed that the 7,6 spiroimine was necessary for binding the muscle and neuronal nAChRs (Aráoz et al. ( 2011) **[8]**)**.** On the other hand, synthetic *gymnodimine-inspired* 6,6 spiroimines were able to inhibit acetylcholine-evoked currents in *Xenopus laevis* oocytes expressing muscle and neuronal nAChR subtypes (Duroure et al. (2011) 6,6-Spiroimine analogs of (-)-gymnodimine A: synthesis and biological evaluation on nicotinic acetylcholine receptors. Organic & Biomolecular Chemistry, 9(23):8112-8118 **[40]**). The macrocycle of gymnodimine was however unable to bind the muscle-type nAChR (Toumieux et al. (2011) Synthesis of the gymnodimine tetrahydrofuran core through a Ueno-Stork radical cyclization. Organic & Biomolecular Chemistry, 9(10):3726-3732 **[41]**). Moreover, the co-crystallization of AChBP with 6,6 synthetic spiroimines, confirmed their interaction with key amino acid residues within the loop C of the ACh-binding pocket virtually occupying the same position as the 6,6 spiroimine of *gymnodimine* (*Bourne et al. submitted*)*.* However, the affinity of the synthetic spiroimines was 3-orders of magnitude lower than the parental *gymnodimine*-A, suggesting that the bulky macrocycle is needed for a better positioning and anchoring of the spiroimine in the ACh-binding site of nAChRs.

Ingrilimine is a potent cytotoxic and apoptosis inducer and like no other CiTX, it possesses a dual activity (antagonist/ agonist) on human neuronal $\alpha$7 nAChR. The less decorated 6,6 spiroimine and its lighter circular macrocycle may allow ingrilimine to interact with the loop C of the acetylcholine-binding pocket of $\alpha$7 nAChR sending some projections to interact with the nearby loop F that governs agonist behavior of nAChRs.

## REFERENCES

**[1]** Aráoz R, Barnes P, Sechet V, Delepierre M, Zinn-Justin S, Molgó J, Zakarian A, Hess P, Servent D. (2020) Cyclic imine toxins survey in coastal european shellfish samples: Bioaccumulation and mode of action of 28-O-palmitoyl ester of pinnatoxin-G. first report of portimine-A bioaccumulation, Harmful Algae, 98.

**[2]** Torigoe K. Murata M. Yasumoto T, Iwashita T. (1988) Prorocentrolide, a toxic nitrogenous macrocycle from a marine dinoflagellate, Prorocentrum-lima., Journal of the American Chemical Society, 110: 7876-7877.

**[3]** Lamoise C, Gaudin A, Hess P, Séchet V, Thai R, Servent D, Zinn-Justin S, Aráoz R. (2017) Physico-chemical and functional characterization of Portimine purified from Vulcanodinium rugosum strain IFR-VRU-01. In Marine and Fresh-Water Harmful Algae. Proceedings of the 17th International Conference on Harmful Algae (Proença, L.A.O.

and Hallegraeff, G. eds) pp. 126-129 ISSHA & IOC/UNESCO, Florianapolis.

**[4]** Molgó J, Marchot P, Aráoz R, Benoit E, Iorga BI, Zakarian A, Taylor P, Bourne Y, Servent D. (2017) Cyclic imine toxins from dinoflagellates: a growing family of potent antagonists of the nicotinic acetylcholine receptors. Journal of Neurochemistry, 142:41-51.

**[5]** Aráoz R, Servent D, Molgó J, Iorga BI, Fruchart-Gaillard C, Benoit E, Gu Z, Stivala C, Zakarian A. (2011) Total Synthesis of Pinnatoxins A and G and Revision of the Mode of Action of Pinnatoxin A. Journal of the American Chemical Society, 133(27):10499-10511.

**[6]** Stivala CE, Benoit E, Aráoz R, Servent D, Novikov A, Molgó J, Zakarian A. (2015) Synthesis and biology of cyclic imine toxins, an emerging class of potent, globally distributed marine toxins. Natural Product Reports, 32(3):411-435.

**[7]** Albuquerque EX, Pereira EFR, Alkondon M, Rogers SW. (2009) Mammalian Nicotinic Acetylcholine Receptors: From Structure to Function. Physiological Reviews, 89(1):73-120.

**[8]** Taly A, Corringer PJ, Guedin D, Lestage P, Changeux JP. (2009) Nicotinic receptors: allosteric transitions and therapeutic targets in the nervous system. Nature reviews Drug discovery, 8(9):733-750.

**[9]** Mukund K, Subramaniam S. (2020) Skeletal muscle: A review of molecular structure and function, in health and disease. Wiley Interdisciplinary Reviews-Systems Biology and Medicine, 12(1).

**[10]** Bertrand D, Lee CH, Flood D, Marger F, Donnelly-Roberts D. (2015) Therapeutic Potential of alpha7 Nicotinic Acetylcholine Receptors. Pharmacological reviews, 67(4): 1025-1073.

**[11]** Dani JA, Bertrand D. (2007) Nicotinic acetylcholine receptors and nicotinic cholinergic mechanisms of the central nervous system. In: Annual Review of Pharmacology and Toxicology, 47: 699-729.

**[12]** Bartus RT. (2000) On neurodegenerative diseases, models, and treatment strategies: Lessons learned and lessons forgotten a generation following the cholinergic hypothesis. Experimental Neurology, 163(2):495-529.

**[13]** McDade E, Bateman RJ. (2017) Stop Alzheimer's before it starts. Nature, 547(7662): 153-155.

**[14]** Wessler I, Kirkpatrick CJ. (2008) Acetylcholine beyond neurons: the non-neuronal cholinergic system in humans. British Journal of Pharmacology, 154(8):1558-1571.

**[15]** Wang SC, Hu Y. (2018) 7 nicotinic acetylcholine receptors in lung cancer. Oncology Letters, 16(2):1375-1382.

**[16]** Resende RR, Adhikari A. (2009) Cholinergic receptor pathways involved in apoptosis, cell proliferation and neuronal differentiation. Cell Communication and Signaling, 7:20.

**[17]** Alonso E, Otero P, Vale C, Alfonso A, Antelo A, Gimenez-Llort L, Chabaud L, Guillou C, Botana LM. (2013) Benefit of 13-desmethyl Spirolide C Treatment in Triple Transgenic Mouse Model of Alzheimer Disease: Beta-Amyloid and Neuronal Markers Improvement. Current Alzheimer Research, 10(3):279-289.

**[18]** Alonso E, Vale C, Vieytes MR, Laferla FM, Gimenez-Llort L, Botana LM. (2011) The cholinergic antagonist gymnodimine improves a beta and tau neuropathology in an in vitro model of Alzheimer Disease. Cellular Physiology and Biochemistry, 27(6):783-794.

**[19]** Botana LM, Alonso E, Vale C. (2011) Use of gymnodimine, analogues and derivatives for the treatment and/or prevention of neurodegenerative diseases associated with Tau and B-amyloid. In: WO2011039394.

**[20]** Botana LM, Alonso E, Vale C. (2012) Use of a spirolide, analogues and derivatives for treating and/or preventing pathological conditions linked to the tau and beta-amyloid proteins. In: US20120258956A1.

**[21]** Boente-Juncal A, Mendez AG, Vale C, Vieytes MR, Botana LM. (2018) In Vitro Effects of Chronic Spirolide Treatment on Human Neuronal Stem Cell Differentiation and Cholinergic System Development. ACS Chemical Neuroscience 2018, 9(6):1441-1452.

**[22]** Hampton MB, Selwood AI, Shi F. (2014) Bioactive compounds. In: WO2014189393.

**[23]** Aráoz R, Ramos S, Pelissier F, Guerineau V, Benoit E, Vilarino N, Botana LM, Zakarian A, Molgó J. (2012) Coupling the Torpedo Microplate-Receptor Binding Assay with Mass Spectrometry to Detect Cyclic Imine Neurotoxins. Analytical Chemistry, 84(23): 10445-10453.

[24] Nyberg NT, Duus JO, Sorensen OW. (2005) Heteronuclear Two-Bond Correlation: Suppressing Heteronuclear Three-Bond or Higher NMR Correlations while Enhancing Two-Bond Correlations even for Vanishing 2JCH. Journal of the American Chemical Society, 127(17):6154-6155.

[25] Hermawan I, Higa M, Hutabarat PUB, Fujiwara T, Akiyama K, Kanamoto A, Haruyama T, Kobayashi N, Higashi M, Suda S, Tanaka J. (2019) Kabirimine, a New Cyclic Imine from an Okinawan Dinoflagellate. Marine Drugs, 17(6).

[26] Seki T, Satake M, Mackenzie L, Kaspar HF, Yasumoto T. (1995) Gymnodimine, a New Marine Toxin of Unprecedented Structure Isolated from New-Zealand Oysters and the Dinoflagellate, Gymnodinium Sp. Tetrahedron Letters, 36(39):7093-7096.

[27] Selwood AI, Wilkins AL, Munday R, Gu H, Smith KF, Rhodes LL, Rise F. (2014) Pinnatoxin H: a new pinnatoxin analogue from a South China Sea Vulcanodinium rugosum isolate. Tetrahedron Letters, 55(40):5508-5510.

[28] Tang JC, Li WC, Chiu TY, Martínez-Peña F, Luo ZW, Chong CT, Wei QJ, Gazaniga N, West TJ, See YY, Lairson LL, Parker CG, Baran PS. (2023) Synthesis of portimines reveals the basis of their anti-cancer activity. Nature 2023, 622(7983):507-513.

[29] Fribley AM, Xi Y, Makris C, Alves-de-Souza C, York R, Tomas C, Wright JLC, Strangman WK. (2019) Identification

of Portimine B, a New Cell Permeable Spiroimine That Induces Apoptosis in Oral Squamous Cell Carcinoma. ACS Medicinal Chemistry

[30] Wang GX, Qiu JB, Li AF, Ji Y, Zhang JR. (2023) Apoptosis and oxidative stress of mouse breast carcinoma 4T1 and human intestinal epithelial Caco-2 cell lines caused by the phycotoxin gymnodimine-A. Chemico-Biological Interactions, 384.

[31] Ly JD, Grubb DR, Lawen A. (2003) The mitochondrial membrane potential ($\Delta\psi m$) in apoptosis; an update. Apoptosis, 8(2):115-128.

[32] Harrington JSS, Ryter SWW, Plataki M, Price DRR, Choi AMK. (2023) Mitochondria in health, disease, and aging. Physiological Reviews, 103(4):2349-2422.

[33] Bourne Y, Sulzenbacher G, Radic Z, Aráoz R, Reynaud M, Benoit E, Zakarian A, Servent D, Molgó J, Taylor P, Marchot P. (2015) Marine Macrocyclic Imines, Pinnatoxins A and G: Structural Determinants and Functional Properties to Distinguish Neuronal $\alpha7$ from Muscle $(\alpha1)2\beta1\gamma\delta$ nAChRs. Structure, 23(6):1106-1115.

[34] Bourne Y, Radic Z, Araoz R, Talley TT, Benoit E, Servent D, Taylor P, Molgó J, Marchot P. (2010) Structural determinants in phycotoxins and AChBP conferring high affinity binding and nicotinic AChR antagonism. Proceedings of the National Academy of Sciences of the United States of America, 107(13):6076-6081.

[35] Aráoz R, Ouanounou G, Iorga BI, Goudet A, Alili D, Amar M, Benoit E, Molgó J, Servent D. (2015) The Neurotoxic Effect of 13,19-Didesmethyl and 13-Desmethyl Spirolide C Phycotoxins Is Mainly Mediated by Nicotinic Rather Than Muscarinic Acetylcholine Receptors. Toxicological Sciences, 147(1):156-167.

**[36]** Couesnon A, Aráoz R, Iorga BI, Benoit E, Reynaud M, Servent D, Molgó J. (2016) The Dinoflagellate Toxin 20-Methyl Spirolide-G Potently Blocks Skeletal Muscle and Neuronal Nicotinic Acetylcholine Receptors. Toxins 2016, 8(9).

**[37]** Amar M, Aráoz R, Iorga BI, Yasumoto T, Servent D, Molgó J. (2018) Prorocentrolide-A from Cultured Prorocentrum lima Dinoflagellates Collected in Japan Blocks Sub-Types of Nicotinic Acetylcholine Receptors. Toxins, 10(3).

**[38]** Hu TM, Curtis JM, Walter JA, Wright JLC. (1996) Characterization of biologically inactive spirolides E and F: Identification of the spirolide pharmacophore. Tetrahedron Letters, 37(43):7671-7674.

**[39]** Stewart M, Blunt JW, Munro MHG, Robinson WT, Hannah DJ (1997) The absolute stereochemistry of the New Zealand shellfish toxin gymnodimine. Tetrahedron Letters, 38(27) :4889-4890.

**[40]** Duroure L, Jousseaume T, Aráoz R, Barre E, Retailleau P, Chabaud L, Molgó J, Guillou C. (2011) 6,6-Spiroimine analogs of (-)-gymnodimine A: synthesis and biological evaluation on nicotinic acetylcholine receptors. Organic & Biomolecular Chemistry, 9(23):8112-8118.

**[41]** Toumieux S, Beniazza R, Desvergnes V, Aráoz R, Molgó J, Landais Y. (2011) Synthesis of the gymnodimine tetrahydrofuran core through a Ueno-Stork radical cyclization. Organic & Biomolecular Chemistry, 9(10):3726-3732.

## Claims

1. Compound of formula (I) or of its pharmaceutically acceptable form selected among salt, prodrug, crystal form, stereoisomer, tautomer, hydrate and solvate thereof:

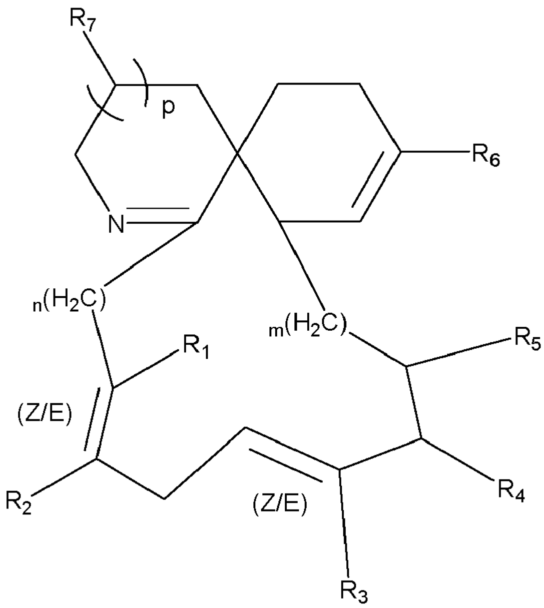

(I)

- wherein the groups $R_1$, $R_2$ and $R_3$ represent independently a hydrogen or an aliphatic chain being at least selected among one of the following: linear, branched, cyclic, substituted and unsubstituted chain; the aliphatic chain being preferably selected among a $C_1$-$C_{12}$ alkyl, and preferably a $C_1$-$C_6$ alkyl such as a methyl, an ethyl or a propyl, and preferably a methyl;
- wherein the groups $R_4$ and $R_5$ represent independently a substituent comprising at least one of the atom or function selectionned from -F; -Cl; -Br; -I; $-NO_2$; -CN; $-CF_3$; -OH and -SH; preferably -OH;
- wherein $R_6$ represents a substituent comprising at least one of the atom or function selected from -F; -Cl; -Br; -I; $-NO_2$; -CN; $-CF_3$; -OH; -SH; thioether; amine; carbonyl; an aliphatic chain selected among a $C_1$-$C_6$ alkyl preferably an alkene group; -C(O)-OH; and an ester;
- wherein the one, or two groups, $R_7$ represent independently a hydrogen or a methyl;
- wherein n and m are integers, independently selected among 1 to 3; and
- wherein p is an integer selected among 1 and 2.

**2.** Compound according to claim 1, wherein the compound is of formula (II):

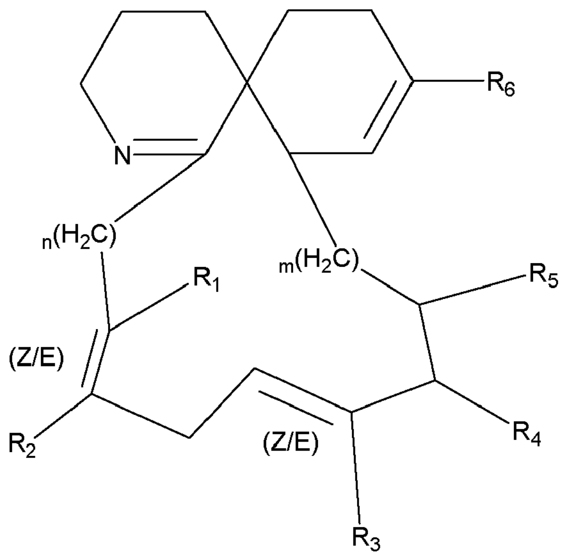

(II)

and wherein the groups $R_1$, $R_2$, $R_3$, $R_4$, $R_5$ and $R_6$; and m, n and p are as defined in claim 1.

**3.** Compound according to claim 1 or 2, wherein the compound is an *ingrilimine* having the following formula (III) :

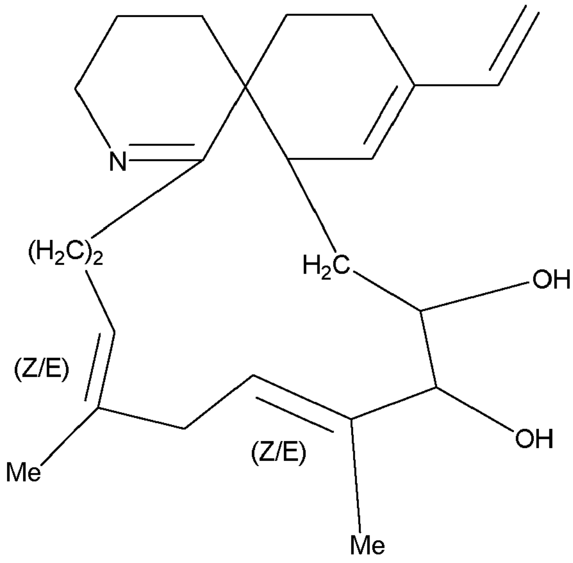

(III)

4. Compound according to claim 3, wherein the compound is selected among at least one of the followings compounds (IIIa) and (IIIb) or a mixture:

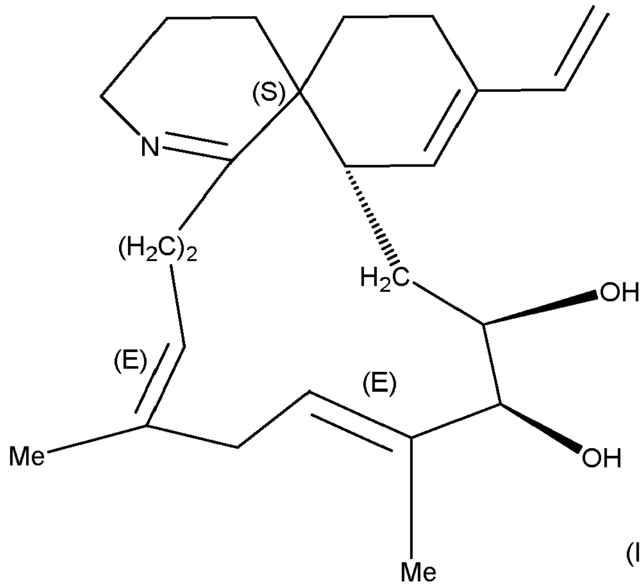

(IIIa)

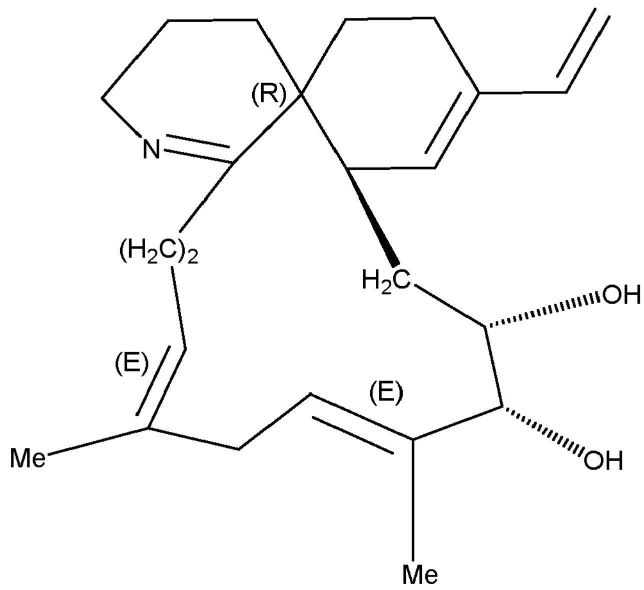

(IIIb)

**5.** Compound according to claim 1 to 3, wherein the double bond bearing the $R_1$ group, preferably a hydrogen, and the $R_2$ group, preferably a methyl, is in the E configuration; and the double bond bearing the $R_3$ group, preferably a methyl, is in the E configuration.

**6.** A compound according to any of claims 1 to 5, for use as a medicament.

**7.** A compound according to any one of claims 1 to 5, for use in the treatment of Chronic Kidney Disease (CDK).

**8.** A compound according to any one of claims 1 to 5, for use in the treatment of at least one of the neurodegenerative diseases selected among Alzheimer disease, Parkinson disease, Schizoprenia, Epiliepsy, Autism and Addiction, preferably Autism.

**9.** A compound according to any one of claims 1 to 5, for use in the treatment of a cancer, preferably lung cancer.

**10.** A compound according to any one of claims 1 to 5, for its use selected among one of the followings: as an $\alpha_7$ nicotinic acetylcholine receptor antagonist and as an $\alpha_7$ nicotinic acetylcholine receptor agonist.

**11.** The compound for its use according to claim 10, wherein the $\alpha_7$ nicotinic acetylcholine receptor is a human $\alpha_7$ nicotinic acetylcholine receptor.

**12.** A pharmaceutical composition comprising the compound according to any one of claims 1 to 5, said compound being dissolved in a nanomolar concentration, and preferably said compound is dissolved in a concentration from 1 to 10 nanomole/L in respect of the total volume of said pharmaceutical composition.

**13.** A pharmaceutical composition comprising the compound according to any one of claims 1 to 5, said compound being dissolved in a micromolar concentration, and preferably said compound is dissolved in a concentration from 1 to 10 micromole/L in respect of the total volume of said pharmaceutical composition.

**14.** The pharmaceutical composition according to any of claims 12 or 13, said composition comprising a pharmaceutically acceptable carrier, preferably an excipient.

**Figure 1**

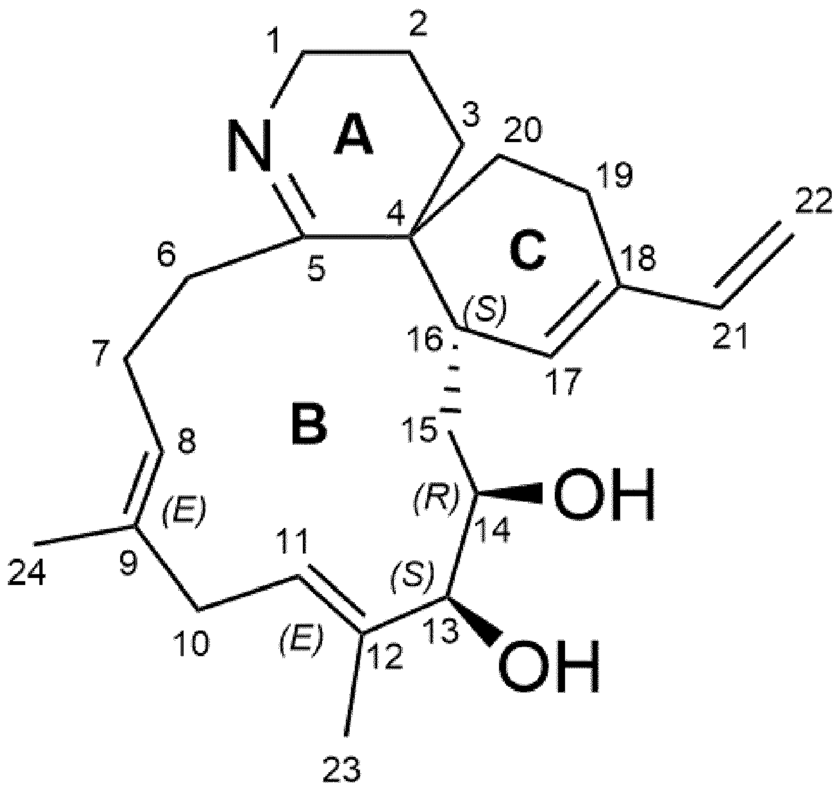

Figure 2

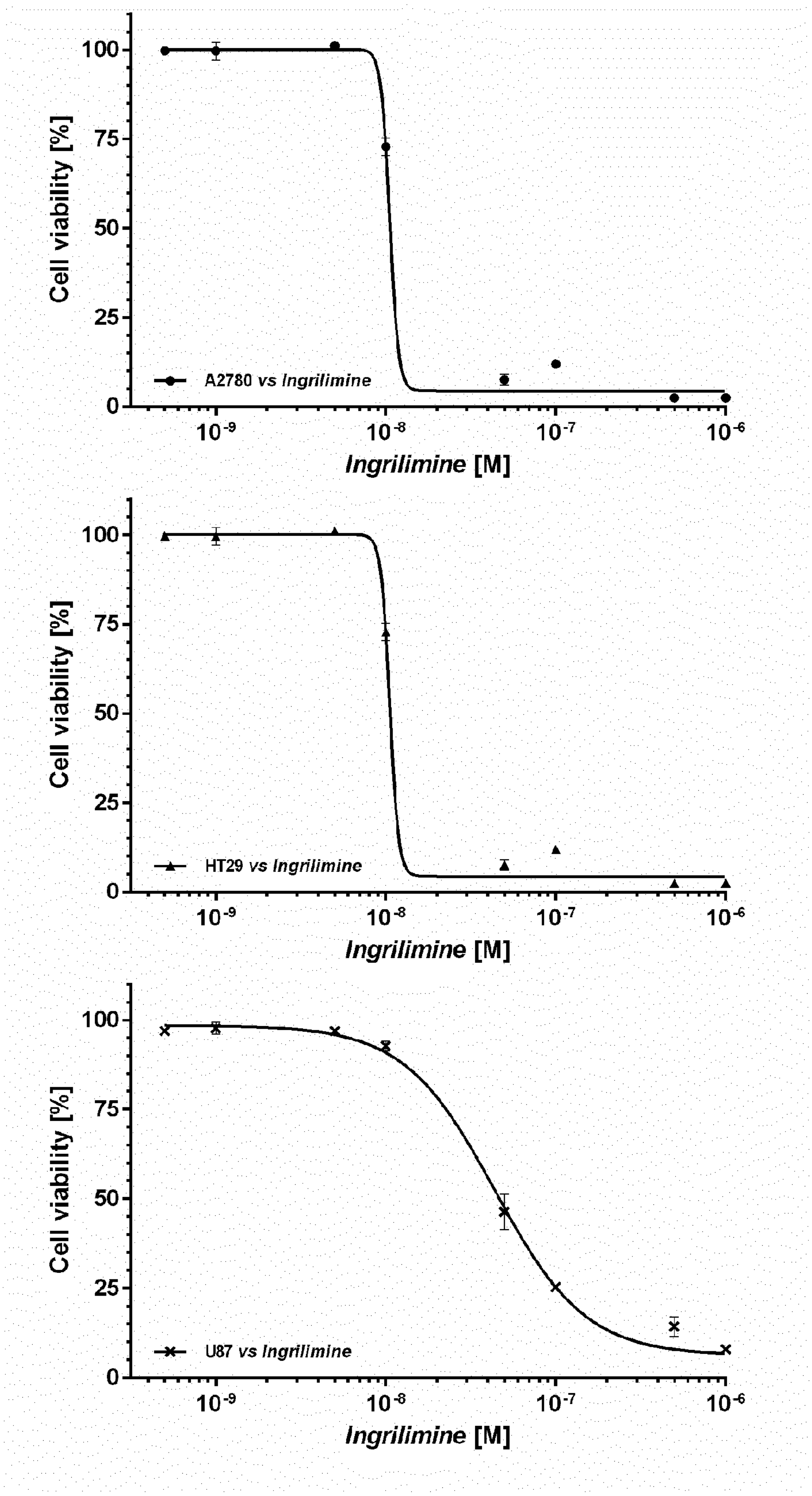

Cell viability [%]
100
75
50
25
0
A2780 vs Ingrilimine
10-9
10-8
10-7
10-6
Ingrilimine [M]
Cell viability [%]
HT29 vs Ingrilimine
Ingrilimine [M]
Cell viability [%]
U87 vs Ingrilimine
Ingrilimine [M]

Figure 3

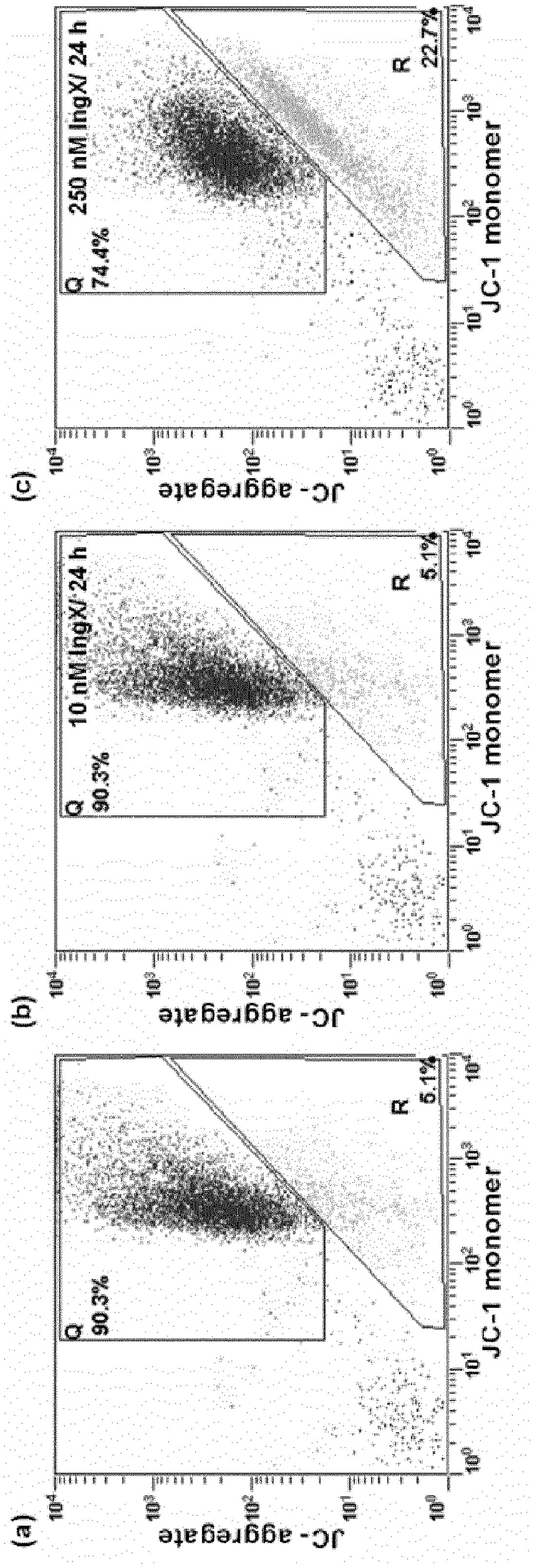
(a)
Q
90.3%
R
5.1%
JC- aggregate
JC-1 monomer
(b)
10 nM IngX/ 24 h
Q
90.3%
R
5.1%
JC- aggregate
JC-1 monomer
(c)
250 nM IngX/ 24 h
Q
74.4%
R
22.7%
JC- aggregate
JC-1 monomer

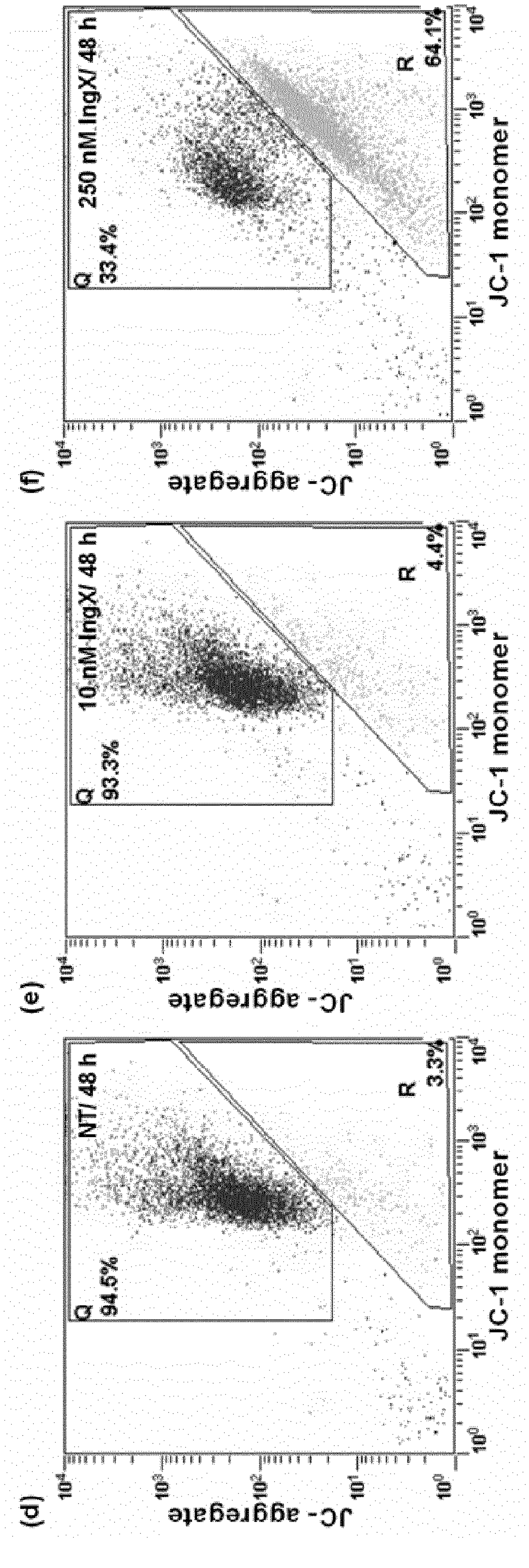
(d)
NT/ 48 h
Q
94.5%
R
3.3%
JC- aggregate
JC-1 monomer
(e)
10 nM IngX/ 48 h
Q
93.3%
R
4.4%
JC- aggregate
JC-1 monomer
(f)
250 nM IngX/ 48 h
Q
33.4%
R
64.1%
JC- aggregate
JC-1 monomer

Figure 4

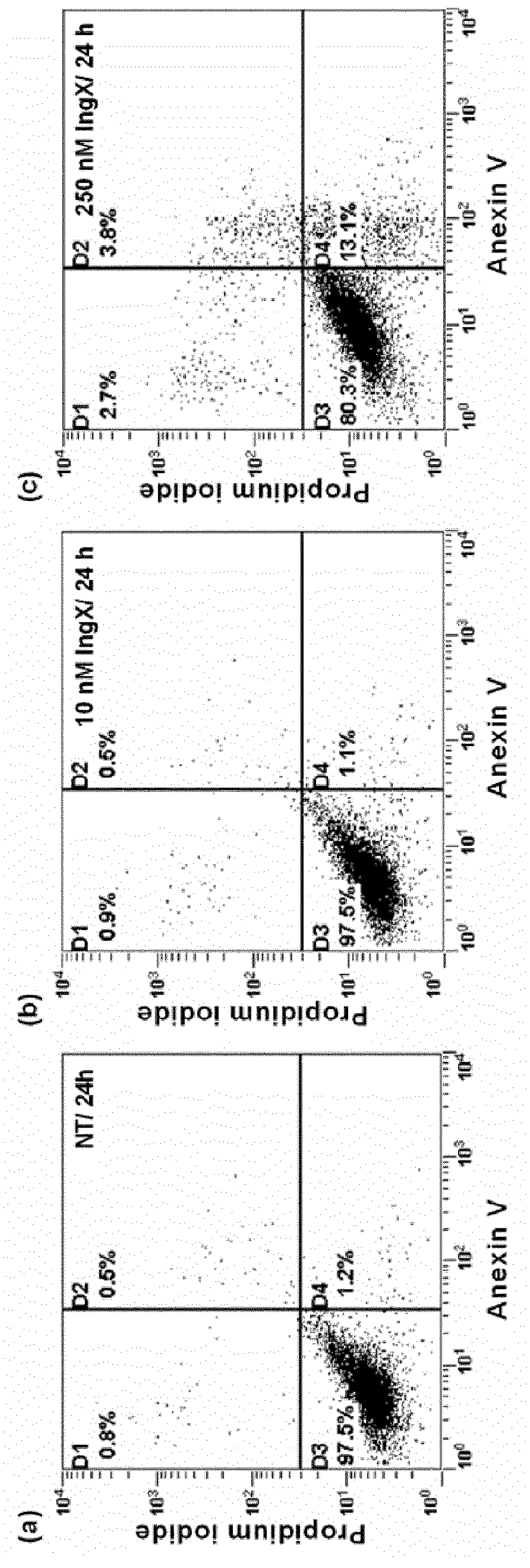
(a)
NT/ 24h
D1 0.8%
D2 0.5%
D3 97.5%
D4 1.2%
(b)
10 nM IngX/ 24 h
D1 0.9%
D2 0.5%
D3 97.5%
D4 1.1%
(c)
250 nM IngX/ 24 h
D1 2.7%
D2 3.8%
D3 80.3%
D4 13.1%
Propidium iodide
Anexin V

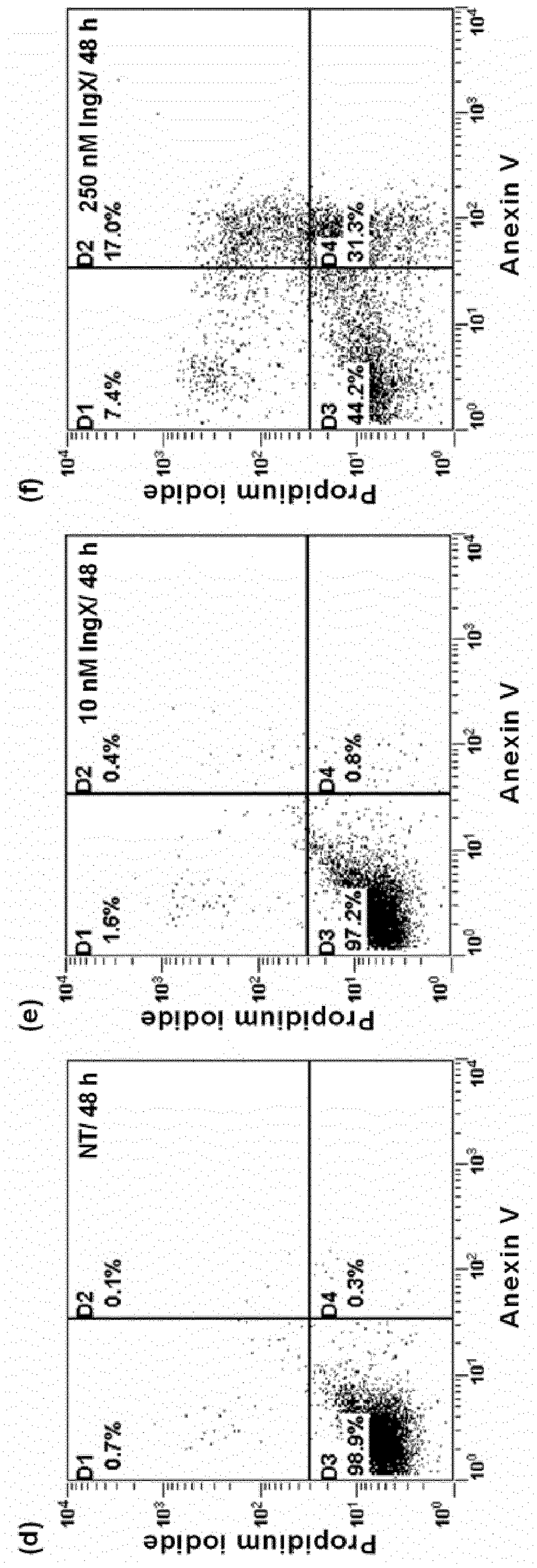
(d)
NT/ 48 h
D1 0.7%
D2 0.1%
D3 98.9%
D4 0.3%
Propidium iodide
Anexin V
(e)
10 nM IngX/ 48 h
D1 1.6%
D2 0.4%
D3 97.2%
D4 0.8%
Propidium iodide
Anexin V
(f)
250 nM IngX/ 48 h
D1 7.4%
D2 17.0%
D3 44.2%
D4 31.3%
Propidium iodide
Anexin V

Figure 5

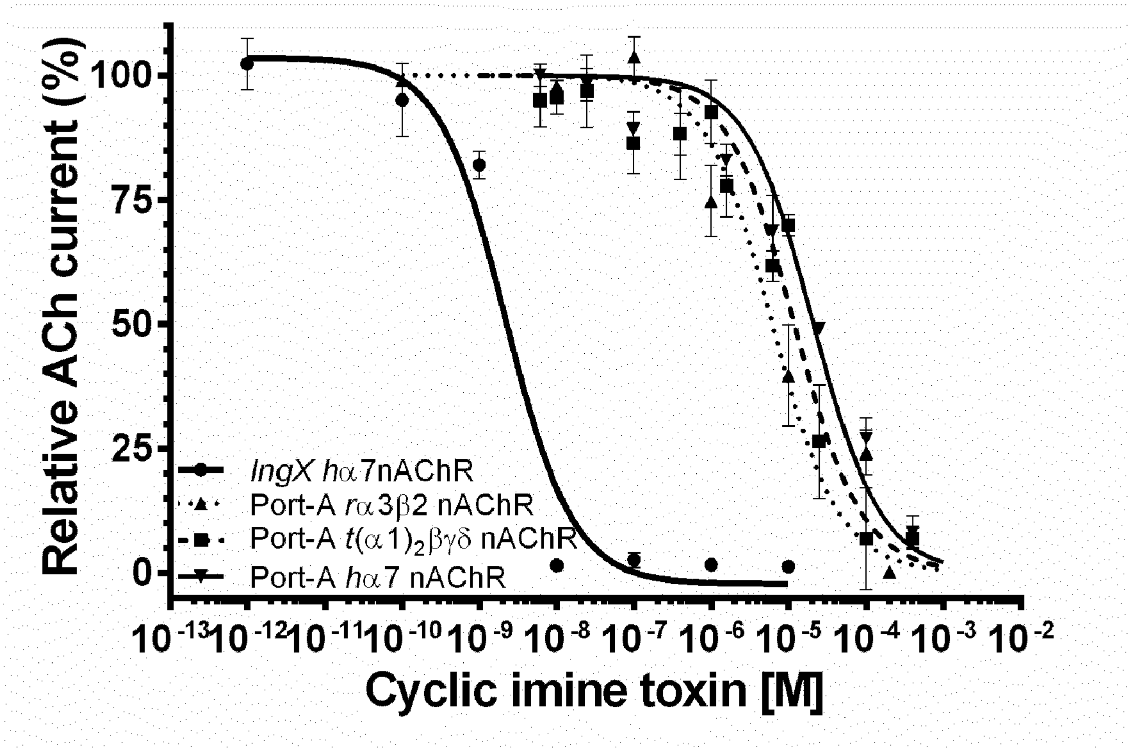
Relative ACh current (%)
100
75
50
25
0
IngX hα7nAChR
Port-A rα3β2 nAChR
Port-A t(α1)2βγδ nAChR
Port-A hα7 nAChR
10 -13 10 -12 10 -11 10 -10 10 -9 10 -8 10 -7 10 -6 10 -5 10 -4 10 -3 10 -2
Cyclic imine toxin [M]

Figure 6

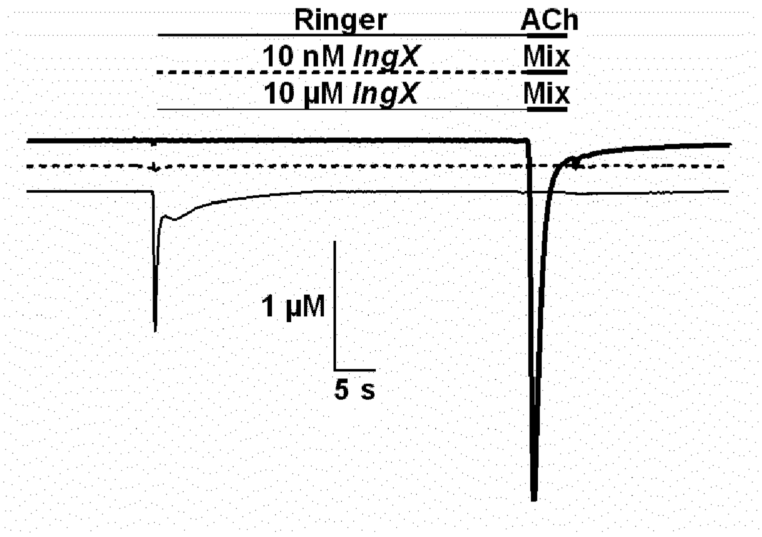

Figure 7

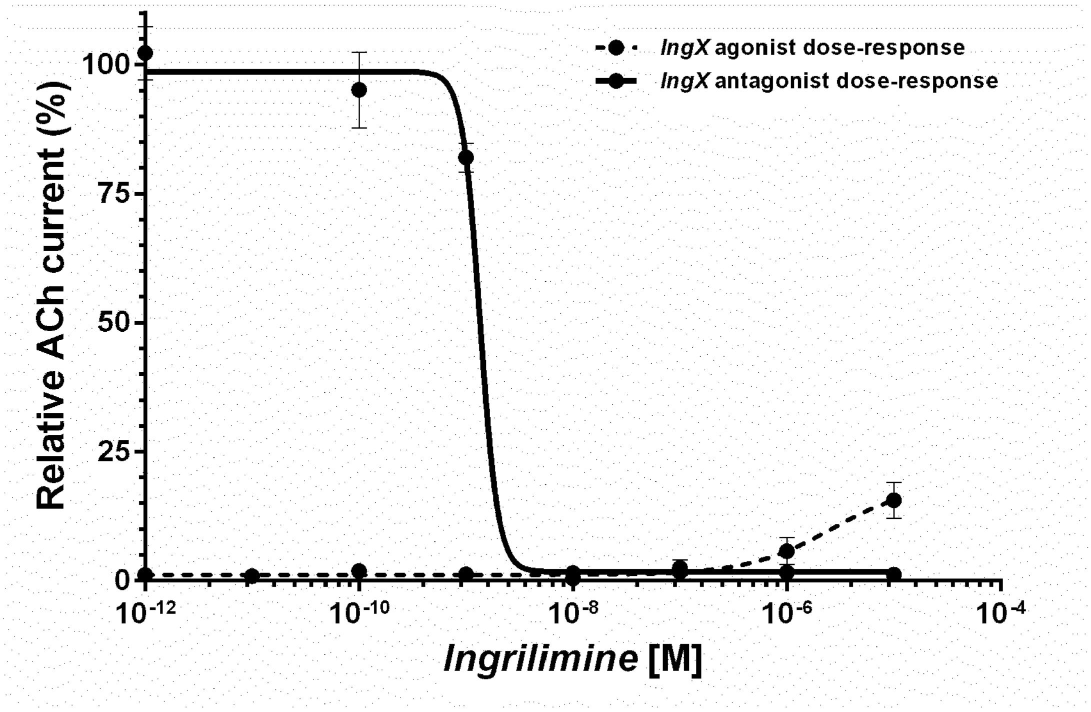
IngX agonist dose-response
IngX antagonist dose-response
Relative ACh current (%)
100
75
50
25
0
10-12
10-10
10-8
10-6
10-4
Ingrilimine [M]

Europäisches Patentamt
European Patent Office
Office européen des brevets

# EUROPEAN SEARCH REPORT

Application Number
EP 24 30 5488

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | STIVALA CRAIG E. ET AL: "Synthesis and biology of cyclic imine toxins, an emerging class of potent, globally distributed marine toxins", NATURAL PRODUCT REPORTS, vol. 32, no. 3, 1 January 2015 (2015-01-01), pages 411-435, XP093201816, GB ISSN: 0265-0568, DOI: 10.1039/C4NP00089G Retrieved from the Internet: URL:https://pubs.rsc.org/en/content/articlepdf/2015/np/c4np00089g> * Chapter 6.2.1; figures 1,2,4,5 * | 1-14 | INV. C07D221/20 A61P13/12 A61P25/00 A61P35/00 A61K31/435 |
| A,D | MOLGÓ JORDI ET AL: "Cyclic imine toxins from dinoflagellates: a growing family of potent antagonists of the nicotinic acetylcholine receptors", JOURNAL OF NEUROCHEMISTRY, vol. 142, no. S2, 21 March 2017 (2017-03-21), pages 41-51, XP093202078, GB ISSN: 0022-3042, DOI: 10.1111/jnc.13995 Retrieved from the Internet: URL:https://api.wiley.com/onlinelibrary/tdm/v1/articles/10.1111%2Fjnc.13995> * page 45, right-hand column, last paragraph - page 47, left-hand column, paragraph first; figure 1; tables 1,2 * | 1-14 | |
| | -/-- | | TECHNICAL FIELDS SEARCHED (IPC) |
| | | | C07D A61P |

The present search report has been drawn up for all claims

2

| Place of search | Date of completion of the search | Examiner |
|---|---|---|
| Munich | 6 September 2024 | Guspanová, Jana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 24 30 5488

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SERVENT DENIS ET AL: "First evidence that emerging pinnatoxin-G, a contaminant of shellfish, reaches the brain and crosses the placental barrier", SCIENCE OF THE TOTAL ENVIRONMENT, ELSEVIER, AMSTERDAM, NL, vol. 790, 31 May 2021 (2021-05-31), XP086719237, ISSN: 0048-9697, DOI: 10.1016/J.SCITOTENV.2021.148125 [retrieved on 2021-05-31] * graphical abstract; abstract; Chapter 3.5 * ----- | 1-14 | |

TECHNICAL FIELDS SEARCHED (IPC)

The present search report has been drawn up for all claims

2

| Place of search | Date of completion of the search | Examiner |
|---|---|---|
| Munich | 6 September 2024 | Guspanová, Jana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011039394 A
- US 20120258956 A1
- WO 2014189393 A, Hampton


### Non-patent literature cited in the description


- **TORIGOE et al.** Prorocentrolide, a toxic nitrogenous macrocycle from a marine dinoflagellate, Prorocentrum-lima.. *Journal of the American Chemical Society*, 1988, vol. 110, 7876-7877
- Physico-chemical and functional characterization of portimine purified from Vulcanodinium rugosum strain IFR-VRU-01. In "Marine and Fresh-Water Harmful Algae. **LAMOISE et al.** Proceedings of the 17th International Conference on Harmful Algae. ISSHA and IOC/UNESCO, 2017, 126-129
- **MOLGÓ et al.** Cyclic imine toxins from dinoflagellates: a growing family of potent antagonists of the nicotinic acetylcholine receptors.. *Journal of Neurochemistry*, 2017, vol. 142, 41-51
- **ARÁOZ et al.** Total synthesis of pinnatoxins A and G and revision of the mode of action of pinnatoxin A.. *Journal of the American Chemical Society*, 2011, vol. 133 (27), 10499-10511
- **LAMOISE** ; **STIVALA et al.** Synthesis and biology of cyclic imine toxins, an emerging class of potent, globally distributed marine toxins.. *Natural Product Reports*, 2015, vol. 32 (3), 411-435
- **ALBUQUERQUE et al.** Mammalian nicotinic acetylcholine receptors: From structure to function. *Physiological Reviews*, 2009, vol. 89 (1), 73-120
- **TALY et al.** Nicotinic receptors: allosteric transitions and therapeutic targets in the nervous system. *Nature reviews Drug discovery*, 2009, vol. 8 (9), 733-750
- **MUKUND et al.** Skeletal muscle: A review of molecular structure and function, in health and disease. *Wiley Interdisciplinary Reviews-Systems Biology and Medicine*, 2020, vol. 12 (1)
- **BERTRAND et al.** Therapeutic potential of alpha7 nicotinic acetylcholine receptors.. *Pharmacological reviews*, 2015, vol. 67 (4), 1025-1073
- **DANI** ; **BERTRAND**. Nicotinic acetylcholine receptors and nicotinic cholinergic mechanisms of the central nervous system.. *Annual Review of Pharmacology and Toxicology*, 2007, vol. 47, 699-729
- **BARTUS**. On neurodegenerative diseases, models, and treatment strategies: Lessons learned and lessons forgotten a generation following the cholinergic hypothesis. *Experimental Neurology*, 2000, vol. 163 (2), 495-529
- **MCDADE** ; **BATEMAN**. Stop Alzheimer's before it starts.. *Nature*, 2017, vol. 547 (7662), 153-155
- **WESSLER** ; **KIRKPATRICK**. Acetylcholine beyond neurons: the non-neuronal cholinergic system in humans.. *British Journal of Pharmacology*, 2008, vol. 154 (8), 1558-1571
- **WANG** ; **HU**. 7 nicotinic acetylcholine receptors in lung cancer. *Oncology Letters*, 2018, vol. 16 (2), 1375-1382
- **RESENDE** ; **ADHIKARI**. Cholinergic receptor pathways involved in apoptosis, cell proliferation and neuronal differentiation.. *Cell Communication and Signaling 2009*, 2009, vol. 7, 20
- **ALONSO et al.** Benefit of 13-desmethyl spirolide C treatment in triple transgenic mouse model of Alzheimer Disease: Beta-Amyloid and neuronal markers improvement.. *Current Alzheimer Research*, 2013, vol. 10 (3), 279-289
- **ALONSO et al.** The cholinergic antagonist gymnodimine improves a beta and tau neuropathology in an in-vitro model of Alzheimer Disease.. *Cellular Physiology and Biochemistry*, 2011, vol. 27 (6), 783-794
- **BOENTE-JUNCAL et al.** effects of chronic spirolide treatment on human neuronal stem cell differentiation and cholinergic system development.. *ACS Chemical Neuroscience*, 2018, vol. 9 (6), 1441-1452
- **ARÁOZ et al.** Coupling the Torpedo Microplate-Receptor Binding Assay with Mass Spectrometry to Detect Cyclic Imine Neurotoxins.. *Analytical Chemistry 2012*, 2012, vol. 84 (23), 10445-10453
- **NYBERG et al.** Heteronuclear Two-Bond Correlation: Suppressing Heteronuclear Three-bond or higher NMR correlations while enhancing two-bond correlations even for vanishing JCH.. *Journal of the American Chemical Society*, 2005, vol. 127 (17), 6154-6155

- **HERMAWAN et al.** Kabirimine, a new cyclic imine from an Okinawan dinoflagellate.. *Marine Drugs*, 2019, vol. 17 (6)
- **SEKI et al.** Gymnodimine, a new marine toxin of unprecedented structure isolated from New-Zealand oysters and the dinoflagellate. *Gymnodinium sp. Tetrahedron Letters*, 1995, vol. 36 (39), 7093-7096
- **SELWOOD et al.** Pinnatoxin H: a new pinnatoxin analogue from a South China Sea Vulcanodinium rugosum isolate. *Tetrahedron Letters*, 2014, vol. 55 (40), 5508-5510
- **TANG et al.** synthesis of portimines reveals the basis of their anti-cancer activity.. *Nature*, 2023, vol. 622 (7983), 507-513
- **FRIBLEY et al.** Identification of portimine B, a new cell permeable spiroimine that induces apoptosis in oral squamous cell carcinoma.. *ACS Medicinal Chemistry Letters*, 2019, vol. 10 (2), 175-179
- **WANG et al.** Apoptosis and oxidative stress of mouse breast carcinoma 4T1 and human intestinal epithelial Caco-2 cell lines caused by the phycotoxin gymnodimine-A.. *Chemico-Biological Interactions*, 2023, vol. 384
- **LY et al.** The mitochondrial membrane potential ($\Delta\psi$m) in apoptosis; an update.. *Apoptosis*, 2003, vol. 8 (2), 115-128
- **HARRINGTON et al.** Mitochondria in health, disease, and aging.. *Physiological Reviews 2023*, 2023, vol. 103 (4), 2349-2422
- **BOURNE et al.** Marine macrocyclic imines, pinnatoxins A and G: Structural determinants and functional properties to distinguish neuronal $\alpha$7 from muscle ($\alpha$1)2$\beta$1$\gamma\delta$ nAChRs.. *Structure 2015*, 2015, vol. 23 (6), 1106-1115
- **BOURNE et al.** Structural determinants in phycotoxins and AChBP conferring high affinity binding and nicotinic AChR antagonism.. *Proceedings of the National Academy of Sciences of the United States of America*, 2010, vol. 107 (13), 6076-6081
- **ARÁOZ et al.** The neurotoxic effect of 13,19-didesmethyl and 13-desmethyl spirolide C phycotoxins is mainly mediated by nicotinic rather than muscarinic acetylcholine receptors.. *Toxicological Sciences*, 2015, vol. 147 (1), 156-167
- **COUESNON et al.** The dinoflagellate toxin 20-methyl spirolide-G potently blocks skeletal muscle and neuronal nicotinic acetylcholine receptors.. *Toxins*, 2016, vol. 8 (9)
- **AMAR et al.** *Prorocentrolide-A from cultured Prorocentrum lima dinoflagellates collected in Japan blocks sub-types of nicotinic acetylcholine receptors.*, 2018, vol. 10 (3), Toxins
- **HU et al.** Characterization of biologically inactive spirolides E and F: Identification of the spirolide pharmacophore.. *Tetrahedron Letters 1996*, 1996, vol. 37 (43), 7671-7674
- **STEWART et al.** The absolute stereochemistry of the New Zealand shellfish toxin gymnodimine.. *Tetrahedron Letters*, 1997, vol. 38 (27), 4889-4890
- **DUROURE et al.** 6,6-Spiroimine analogs of (-)-gymnodimine A: synthesis and biological evaluation on nicotinic acetylcholine receptors.. *Organic & Biomolecular Chemistry*, 2011, vol. 9 (23), 8112-8118
- **TOUMIEUX et al.** Synthesis of the gymnodimine tetrahydrofuran core through a Ueno-Stork radical cyclization.. *Organic & Biomolecular Chemistry*, 2011, vol. 9 (10), 3726-3732
- **ARÁOZ R** ; **BARNES P** ; **SECHET V** ; **DELEPIERRE M** ; **ZINN-JUSTIN S** ; **MOLGÓ J** ; **ZAKARIAN A** ; **HESS P** ; **SERVENT D.** Cyclic imine toxins survey in coastal european shellfish samples: Bioaccumulation and mode of action of 28-O-palmitoyl ester of pinnatoxin-G. first report of portimine-A bioaccumulation. *Harmful Algae*, 2020, vol. 98
- **TORIGOE K.** ; **MURATA M.** ; **YASUMOTO T** ; **IWASHITA T.** Prorocentrolide, a toxic nitrogenous macrocycle from a marine dinoflagellate, Prorocentrum-lima.. *Journal of the American Chemical Society*, 1988, vol. 110, 7876-7877
- Physico-chemical and functional characterization of Portimine purified from Vulcanodinium rugosum strain IFR-VRU-01. **LAMOISE C** ; **GAUDIN A** ; **HESS P** ; **SÉCHET V** ; **THAI R** ; **SERVENT D** ; **ZINN-JUSTIN S** ; **ARÁOZ R.** Marine and Fresh-Water Harmful Algae. Proceedings of the 17th International Conference on Harmful Algae. ISSHA & IOC/UNESCO, 2017, 126-129
- **MOLGÓ J** ; **MARCHOT P** ; **ARÁOZ R** ; **BENOIT E** ; **LORGA BI** ; **ZAKARIAN A** ; **TAYLOR P** ; **BOURNE Y** ; **SERVENT D.** Cyclic imine toxins from dinoflagellates: a growing family of potent antagonists of the nicotinic acetylcholine receptors.. *Journal of Neurochemistry*, 2017, vol. 142, 41-51
- **ARÁOZ R** ; **SERVENT D** ; **MOLGÓ J** ; **LORGA BL** ; **FRUCHART-GAILLARD C** ; **BENOIT E** ; **GU Z** ; **STIVALA C** ; **ZAKARIAN A.** Total Synthesis of Pinnatoxins A and G and Revision of the Mode of Action of Pinnatoxin A.. *Journal of the American Chemical Society*, 2011, vol. 133 (27), 10499-10511
- **STIVALA CE** ; **BENOIT E** ; **ARÁOZ R** ; **SERVENT D** ; **NOVIKOV A** ; **MOLGÓ J** ; **ZAKARIAN A.** Synthesis and biology of cyclic imine toxins, an emerging class of potent, globally distributed marine toxins.. *Natural Product Reports*, 2015, vol. 32 (3), 411-435
- **ALBUQUERQUE EX** ; **PEREIRA EFR** ; **ALKONDON M** ; **ROGERS SW.** Mammalian Nicotinic Acetylcholine Receptors: From Structure to Function.. *Physiological Reviews*, 2009, vol. 89 (1), 73-120
- **TALY A** ; **CORRINGER PJ** ; **GUEDIN D** ; **LESTAGE P** ; **CHANGEUX JP**. Nicotinic receptors: allosteric transitions and therapeutic targets in the nervous system.. *Nature reviews Drug discovery*, 2009, vol. 8 (9), 733-750

- **MUKUND K** ; **SUBRAMANIAM S.** Skeletal muscle: A review of molecular structure and function, in health and disease.. *Wiley Interdisciplinary Reviews-Systems Biology and Medicine*, 2020, vol. 12 (1)
- **BERTRAND D** ; **LEE CH** ; **FLOOD D** ; **MARGER F** ; **DONNELLY-ROBERTS D.** Therapeutic Potential of alpha7 Nicotinic Acetylcholine Receptors.. *Pharmacological reviews*, 2015, vol. 67 (4), 1025-1073
- **DANI JA** ; **BERTRAND D**. Nicotinic acetylcholine receptors and nicotinic cholinergic mechanisms of the central nervous system.. *Annual Review of Pharmacology and Toxicology*, 2007, vol. 47, 699-729
- **BARTUS RT.** On neurodegenerative diseases, models, and treatment strategies: Lessons learned and lessons forgotten a generation following the cholinergic hypothesis.. *Experimental Neurology*, 2000, vol. 163 (2), 495-529
- **MCDADE E** ; **BATEMAN RJ.** Stop Alzheimer's before it starts.. *Nature*, 2017, vol. 547 (7662), 153-155
- **WESSLER I** ; **KIRKPATRICK CJ.** Acetylcholine beyond neurons: the non-neuronal cholinergic system in humans.. *British Journal of Pharmacology*, 2008, vol. 154 (8), 1558-1571
- **WANG SC** ; **HU Y.** 7 nicotinic acetylcholine receptors in lung cancer.. *Oncology Letters*, 2018, vol. 16 (2), 1375-1382
- **RESENDE RR** ; **ADHIKARI A.** Cholinergic receptor pathways involved in apoptosis, cell proliferation and neuronal differentiation.. *Cell Communication and Signaling*, 2009, vol. 7, 20
- **ALONSO E** ; **OTERO P** ; **VALE C** ; **ALFONSO A** ; **ANTELO A** ; **GIMENEZ-LLORT L** ; **CHABAUD L** ; **GUILLOU C** ; **BOTANA LM.** Benefit of 13-desmethyl Spirolide C Treatment in Triple Transgenic Mouse Model of Alzheimer Disease: Beta-Amyloid and Neuronal Markers Improvement.. *Current Alzheimer Research*, 2013, vol. 10 (3), 279-289
- **ALONSO E** ; **VALE C** ; **VIEYTES MR** ; **LAFERLA FM** ; **GIMENEZ-LLORT L** ; **BOTANA LM.** The cholinergic antagonist gymnodimine improves a beta and tau neuropathology in an in vitro model of Alzheimer Disease.. *Cellular Physiology and Biochemistry*, 2011, vol. 27 (6), 783-794
- **BOENTE-JUNCAL A** ; **MENDEZ AG** ; **VALE C** ; **VIEYTES MR** ; **BOTANA LM**. Effects of Chronic Spirolide Treatment on Human Neuronal Stem Cell Differentiation and Cholinergic System Development.. *ACS Chemical Neuroscience 2018*, 2018, vol. 9 (6), 1441-1452
- **HAMPTON MB** ; **SELWOOD AL** ; **SHI F.** *Bioactive compounds.*, 2014
- **ARÁOZ R** ; **RAMOS S** ; **PELISSIER F** ; **GUERINEAU V** ; **BENOIT E** ; **VILARINO N** ; **BOTANA LM** ; **ZAKARIAN A** ; **MOLGÓ J.** Coupling the Torpedo Microplate-Receptor Binding Assay with Mass Spectrometry to Detect Cyclic Imine Neurotoxins.. *Analytical Chemistry*, 2012, vol. 84 (23), 10445-10453
- **NYBERG NT** ; **DUUS JO** ; **SORENSEN OW.** Heteronuclear Two-Bond Correlation: Suppressing Heteronuclear Three-Bond or Higher NMR Correlations while Enhancing Two-Bond Correlations even for Vanishing JCH.. *Journal of the American Chemical Society*, 2005, vol. 127 (17), 6154-6155
- **HERMAWAN I** ; **HIGA M** ; **HUTABARAT PUB** ; **FUJIWARA T** ; **AKIYAMA K** ; **KANAMOTO A** ; **HARUYAMA T** ; **KOBAYASHI N** ; **HIGASHI M** ; **SUDA S**. Kabirimine, a New Cyclic Imine from an Okinawan Dinoflagellate. *Marine Drugs*, 2019, vol. 17 (6)
- **SEKI T** ; **SATAKE M** ; **MACKENZIE L** ; **KASPAR HF** ; **YASUMOTO T.** Gymnodimine, a New Marine Toxin of Unprecedented Structure Isolated from New-Zealand Oysters and the Dinoflagellate. *Gymnodinium Sp. Tetrahedron Letters*, 1995, vol. 36 (39), 7093-7096
- **SELWOOD AL** ; **WILKINS AL** ; **MUNDAY R** ; **GU H** ; **SMITH KF** ; **RHODES LL** ; **RISE F.** Pinnatoxin H: a new pinnatoxin analogue from a South China Sea Vulcanodinium rugosum isolate.. *Tetrahedron Letters*, 2014, vol. 55 (40), 5508-5510
- **TANG JC** ; **LI WC** ; **CHIU TY** ; **MARTÍNEZ-PEÑA F** ; **LUO ZW** ; **CHONG CT** ; **WEI QJ** ; **GAZANIGA N** ; **WEST TJ** ; **SEE YY**. Synthesis of portimines reveals the basis of their anti-cancer activity.. *Nature 2023*, 2023, vol. 622 (7983), 507-513
- **FRIBLEY AM** ; **XI Y** ; **MAKRIS C** ; **ALVES-DE-SOUZA C** ; **YORK R** ; **TOMAS C** ; **WRIGHT JLC** ; **STRANGMAN WK**. Identification of Portimine B, a New Cell Permeable Spiroimine That Induces Apoptosis in Oral Squamous Cell Carcinoma. *ACS Medicinal Chemistry*, 2019
- **WANG GX** ; **QIU JB** ; **LI AF** ; **JI Y** ; **ZHANG JR.** Apoptosis and oxidative stress of mouse breast carcinoma 4T1 and human intestinal epithelial Caco-2 cell lines caused by the phycotoxin gymnodimine-A.. *Chemico-Biological Interactions*, 2023, vol. 384
- **LY JD** ; **GRUBB DR** ; **LAWEN A.** The mitochondrial membrane potential ($\Delta\psi$m) in apoptosis; an update.. *Apoptosis*, 2003, vol. 8 (2), 115-128
- **HARRINGTON JSS** ; **RYTER SWW** ; **PLATAKI M** ; **PRICE DRR** ; **CHOI AMK**. Mitochondria in health, disease, and aging. *Physiological Reviews*, 2023, vol. 103 (4), 2349-2422

- **BOURNE Y** ; **SULZENBACHER G** ; **RADIC Z** ; **ARÁOZ R** ; **REYNAUD M** ; **BENOIT E** ; **ZAKARIAN A** ; **SERVENT D** ; **MOLGÓ J** ; **TAYLOR P**. Marine Macrocyclic Imines, Pinnatoxins A and G: Structural Determinants and Functional Properties to Distinguish Neuronal α7 from Muscle (α1)2β1γδ nAChRs. *Structure*, 2015, vol. 23 (6), 1106-1115
- **BOURNE Y** ; **RADIC Z** ; **ARAOZ R** ; **TALLEY TT** ; **BENOIT E** ; **SERVENT D** ; **TAYLOR P** ; **MOLGÓ J** ; **MARCHOT P.** Structural determinants in phycotoxins and AChBP conferring high affinity binding and nicotinic AChR antagonism.. *Proceedings of the National Academy of Sciences of the United States of America*, 2010, vol. 107 (13), 6076-6081
- **ARÁOZ R** ; **OUANOUNOU G** ; **LORGA BI** ; **GOUDET A** ; **ALILI D** ; **AMAR M** ; **BENOIT E** ; **MOLGÓ J** ; **SERVENT D**. The Neurotoxic Effect of 13,19-Didesmethyl and 13-Desmethyl Spirolide C Phycotoxins Is Mainly Mediated by Nicotinic Rather Than Muscarinic Acetylcholine Receptors.. *Toxicological Sciences*, 2015, vol. 147 (1), 156-167
- **COUESNON A** ; **ARÁOZ R** ; **LORGA BI** ; **BENOIT E** ; **REYNAUD M** ; **SERVENT D** ; **MOLGÓ J.** The Dinoflagellate Toxin 20-Methyl Spirolide-G Potently Blocks Skeletal Muscle and Neuronal Nicotinic Acetylcholine Receptors.. *Toxins 2016*, 2016, vol. 8 (9)
- **AMAR M** ; **ARÁOZ R** ; **LORGA BI** ; **YASUMOTO T** ; **SERVENT D** ; **MOLGÓ J.** Prorocentrolide-A from Cultured Prorocentrum lima Dinoflagellates Collected in Japan Blocks Sub-Types of Nicotinic Acetylcholine Receptors.. *Toxins*, 2018, vol. 10 (3)
- **HU TM** ; **CURTIS JM** ; **WALTER JA** ; **WRIGHT JLC.** Characterization of biologically inactive spirolides E and F: Identification of the spirolide pharmacophore.. *Tetrahedron Letters*, 1996, vol. 37 (43), 7671-7674
- **STEWART M** ; **BLUNT JW** ; **MUNRO MHG** ; **ROBINSON WT** ; **HANNAH DJ**. The absolute stereochemistry of the New Zealand shellfish toxin gymnodimine.. *Tetrahedron Letters*, 1997, vol. 38 (27), 4889-4890
- **DUROURE L** ; **JOUSSEAUME T** ; **ARÁOZ R** ; **BARRE E** ; **RETAILLEAU P** ; **CHABAUD L** ; **MOLGÓ J** ; **GUILLOU C.** 6,6-Spiroimine analogs of (-)-gymnodimine A: synthesis and biological evaluation on nicotinic acetylcholine receptors.. *Organic & Biomolecular Chemistry*, 2011, vol. 9 (23), 8112-8118
- **TOUMIEUX S** ; **BENIAZZA R** ; **DESVERGNES V** ; **ARÁOZ R** ; **MOLGÓ J** ; **LANDAIS Y.** Synthesis of the gymnodimine tetrahydrofuran core through a Ueno-Stork radical cyclization.. *Organic & Biomolecular Chemistry*, 2011, vol. 9 (10), 3726-3732